# EUROPÄISCHE PATENTSCHRIFT

(11) **EP 1 412 022 B1**
(45) Veröffentlichungstag und Bekanntmachung des Hinweises auf die Patenterteilung: **13.04.2005**
(21) Anmeldenummer: 02754953.4
(22) Anmeldetag: 29.07.2002
(51) Int. Cl.: A61N 1/36

(54) **NEUROSTIMULATIONSEINHEIT ZUR IMMOBILISATION DES HERZENS WÄHREND KARDIOCHIRURGISCHER OPERATIONEN**
NEUROSTIMULATION UNIT FOR IMMOBILIZING THE HEART DURING CARDIOSURGICAL OPERATIONS
UNITE DE NEUROSTIMULATION PERMETTANT D'IMMOBILISER LE COEUR AU COURS D'UNE OPERATION DE CHIRURGIE CARDIAQUE

(30) Priorität: 27.07.2001 DE 10136567
(43) Veröffentlichungstag der Anmeldung: 28.04.2004
(73) Patentinhaber: Impella Cardiotechnik AG, 52074 Aachen (DE)
(72) Erfinder: SCHAUERTE, Patrick, 52066-Aachen (DE)
(74) Vertreter: Karlhuber, Mathias
(86) Internationale Anmeldenummer: PCT/EP2002/008443
(87) Internationale Veröffentlichungsnummer: WO 2003/011388

(56) Entgegenhaltungen:
- WO-A-00/72912
- US-A- 5 913 876
- US-A- 6 006 134

## Beschreibung

Die Erfindung betrifft eine Stimulationsvorrichtung, mit der selektiv epikardiale oder extrakardiale parasympathische Herznerven, die den Sinusknoten oder den atrioventrikulären Knoten innervieren, elektrisch stimuliert werden können, um durch eine Herzschlagfrequenzminderung das Herz während einer Herzoperation weitestgehend elektrisch zu immobilisieren.

Die Kreislaufunterstützung während einer Herzoperation mittels der extrakorporalen Zirkulation der Herz-Lungen Maschine hat spezifische Nachteile. Kritisch ist insbesondere das Auftreten von systemischen Entzündungs-/Immunreaktionen, die thrombogene Wirkung von Fremdmaterial, das postoperativ vor allem durch die Kalium-Kardioplegie verminderte Herzzeitvolumen (low output state) sowie die geänderte Strömungsdynamik unter künstlichen Kreislaufbedingungen (Cremer et al. Ann Thorac Surg 1996;61:1714-20; Myles et al. Med J Austr 1993;158:675-7; Roach et al. N Engl J Med;335:1857-63; McKhan GM et al. Ann Thorac Surg 1997;63:516-521; Ede et al. Ann Thorac Surg 1997;63:721-7). Die Verminderung der Perfusion aller Körperorgane insbesondere während der Operation, die durch viele dieser Faktoren beeinflusst wird, kann zu bleibenden Organschäden, z.B. neurophysiologischen und neuropsychologischen Schäden bis hin zum Schlaganfall oder z.B. zum Nierenversagen führen (Cremer et al. Ann Thorac Surg 1996;61:1714-20; Myles et al. Med J Austr 1993;158:675-7; Roach et al. N Engl J Med;335:1857-63; McKhan GM et al. Ann Thorac Surg 1997;63:516-521).

Daher wurden Methoden entwickelt, mit Hilfe derer ein Bypass auf die Herzkranzgefäße angelegt werden kann, ohne dass eine Kreislaufunterstützung während einer Herzoperation mittels der extrakorporalen Zirkulation der Herz-Lungen Maschine erfolgen muss.

Lokale Stabilisatoren des myokardialen Areals in unmittelbarer Nähe eines Herzkranzgefäßes ermöglichen beispielweise die Anlage der koronaren Anastomose am schlagenden Herzen (Boonstra et al. Ann Thorac Surg 1997;63:567-9).

Die Herz-Operation mit Kreislaufunterstützung durch Mikroaxialpumpen ist eine weitere schonende Alternative zu Operationen mit Hilfe der Herz-Lungen-Maschine. Mikroaxialpumpen zur Herzunterstützung sind seit langem bekannt. Sie besitzen ein die Blutströmung unterstützendes Flügelrad, welches häufig als Rotor oder Impella bezeichnet wird und um eine koaxial im Blutgefäß angeordnete Achse rotiert. In EP 0 157 871 B1 und EP 0 397 668 B1 wird eine Mikroaxialpumpe beschrieben, bei der das Flügelrad über eine flexible Welle, die durch den Katheter hindurch verläuft, mit einem extrakorporalen Antriebsteil verbunden ist. Der Antriebsteil treibt die flexible Welle an, die ihrerseits das Flügelrad der Mikroaxialpumpe antreibt.

Jüngere Entwicklungen der Mikroaxialpumpen, die sich auch bereits im klinischen Einsatz befinden, sehen vor, den Antriebsteil und den Pumpenteil in einer Einheit zusammenzufassen und als Einheit in das Gefäßsystem des Körpers zu implantieren. Anstelle der mechanisch anfälligen Antriebswelle verläuft durch den Katheter lediglich noch ein Versorgungskabel zur Versorgung des Antriebsteils mit elektrischer Energie. Eine solche Mikroaxialpumpe, allgemein auch als "intravasale Blutpumpe" bezeichnet, ist beispielsweise in der DE 196 13 564 Cl beschrieben.

Ein aus zwei Pumpen bestehendes Pumpsystem kann zur teiloder vollständigen Übernahme der Pumpfunktion des Herzen eingesetzt werden (beschrieben in PCT/EP/98/01868). Solch ein System kann ein Herzzeitvolumen von etwa 4,5 Liter Blut pro Minute übernehmen und eine vermehrte Wandexkursion während z.B. einer Bradykardie verringern. Die Pumpvorrichtung weist eine erste Pumpe auf, die mit ihrer Saugseite in den linken Ventrikel des Herzens eingeführt werden kann, während eine zweite Pumpe, die mit ihrer Saugseite im rechten Vorhof angeordnet wird, mit ihrer Druckseite in der Pulmonalarterie liegt. Beide Pumpen werden von einer gemeinsamen Steuereinheit betrieben. Beide Pumpen werden in das Herz eingeführt, ohne das die Ventrikel geöffnet werden müssen.

Eine sogenannte "parakardiale Blutpumpe" (Deutsche Patentanmeldung 100 16 422.6-35) kann darüber hinaus ein noch höheres Herzzeitvolumen von fünf bis sechs Litern Blut pro Minute übernehmen und damit die bei Bradykardie auftretende vermehrte Wandexkursion, bedingt durch die komplette Übernahme der Herzfunktion durch die Pumpe, minimieren. Im Gegensatz zur beschriebenen "intravasalen Pumpe" ist dies eine Blutpumpe, die Blut aus einem Teil des Herzens ansaugt und in die Aorta oder ein anderes Zielgebiet fördert, wobei das Gehäuse der Blutpumpe außen an das Herz angelegt wird, während der Pumpeinlass eine direkte Verbindung zu derjenigen Herzkammer hat, aus der angesaugt wird.

Wird durch den Einsatz der oben beschriebenen intra- oder parakardialen Pumpe das gesamte Herzzeitvolumen während stark ausgeprägter Bradykardie übernommen, so erfolgt die Flüssigkeitsbalance mittels der in die Pumpen integrierten Sensorik, die eine Kontrolle des geförderten Blutflusses in gegenseitiger Abhängigkeit der Pumpen erlaubt.

Im Gegensatz zur Operation mit der Herz-Lungen-Maschine schlägt -trotz Übernahme des Herzzeitvolumens durch die Blutpumpe- das Herz aufgrund seiner fortbestehenden elektrischen Eigenaktivität noch, ohne allerdings ein relevantes Blutvolumen zu fördern. Diese mechanische Bewegung erschwert die Operation am offenen Herzen und erhöht den myokardialen Sauerstoffverbrauch.

Um diese Bewegung des Herzens während einer Operation zu minimieren, ist im Folgenden eine erfindungsgemäße Stimulationsvorrichtung beschrieben, mit der selektiv epikardiale oder extrakardiale parasympathische Herznerven, die den Sinusknoten oder den atrioventrikulären Knoten innervieren, elektrisch stimuliert werden können, um durch eine Herzschlagfrequenzminderung das Herz während einer Herzoperation weitestgehend elektrisch zu immobilisieren. Insbesondere ist die Kombination einer derartigen Neurostimulationsvorrichtung mit intra- oder parakardialen Blutpumpen beschrieben, um die Perfusion der Organe einschließlich des Herzens während einer Bradykardie zu gewährleisten.

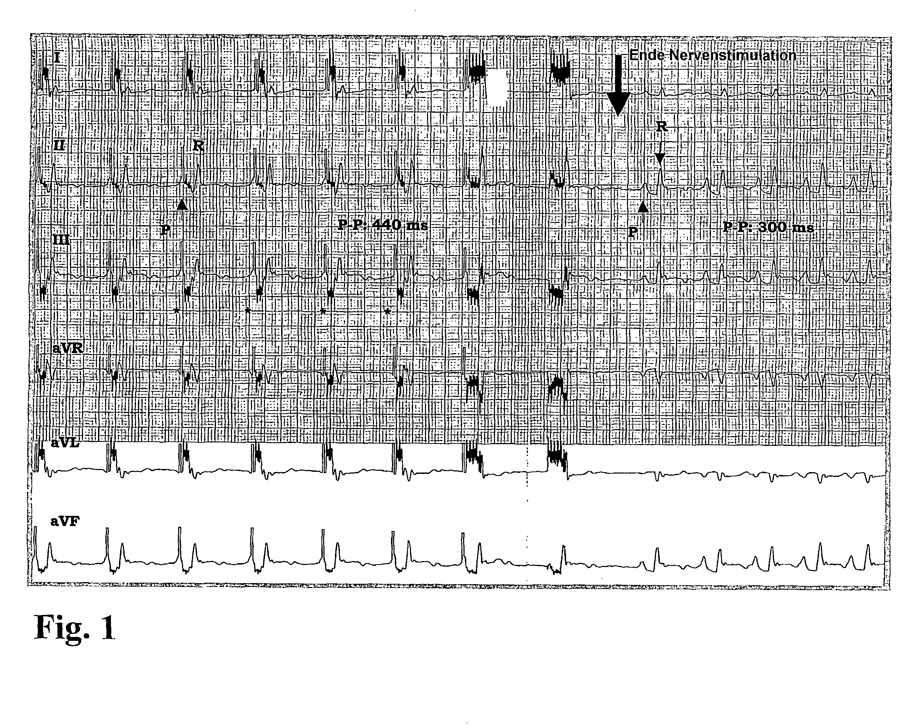

Am gesunden Herzen wir die spontane Herzfrequenz durch die Impulserzeugungsrate des Schrittmacherzentrums des Herzens, des sogenannten Sinusknotens bestimmt. Der Sinusknoten liegt am lateralen hohen rechten Vorhof. Die elektrische Überleitung der Erregung von den Vorhöfen auf die Herzkammern wiederum erfolgt über den sogenannten atrioventrikulären (AV) Knoten. Das vegetative autonome Nervensystem besteht aus einem erregenden Teil, dem Sympathikus, und einem beruhigenden Teil, dem Parasympathikus. Eine Aktivierung des parasympathischen Nervensystems führt zu einer Verlangsamung der Sinusknotenfrequenz (negativ chronotrope Wirkung) sowie zu einer Verzögerung der atrioventrikulären Überleitung über den AV Knoten (negativ dromotrope Wirkung). Parasympathische Nerven, die den Sinus- und den AV Knoten innervieren, verlaufen extrakardial entlang der oberen Hohlvene und entlang den Pulmonalarterien zum Sinusknoten oder AV Knoten, um dann in der Nähe des Zielorgans in umschriebenen epikardialen Fett-Bindegewebsansammlungen zu akkumulieren (sogenannte Nervenplexus oder "fat pads"). Der Nervenplexus, der nahezu alle parasympathischen Fasern, die den Sinusknoten innervieren, enthält, liegt epikardial am lateralen rechten Vorhof im Winkel zwischen rechter Vorhofwand und den die rechte Vorhofwand hinterkreuzenden rechtsseitigen Pulmonalvenen (sogenannter ventraler rechtsatrialer Plexus). Der Nervenplexus, der die überwiegende Anzahl von parasympathischen Nervenfasern enthält, die den atrioventrikulären Knoten innervieren, enthält befindet sich im Winkel zwischen dem Koronarsinusostium, der unteren Hohlvene und dem linken Vorhof (sogenannter inferiorer interatrialer Plexus). Abbildung 1 zeigt eine schematische Darstellung dieser parasympathischen Nervenplexus.

Die epikardiale elektrische Stimulation des ventralen rechtsatrialen Plexus löst eine Sinusbradykardie aus ohne jedoch relevant die AV Knoten Überleitung zu beeinflussen. Eine epikardiale oder transvaskuläre elektrische Stimulation des inferioren interatrialen Plexus verlangsamt die atrioventrikuläre Überleitung (Schauerte P et al. Catheter stimulation of cardiac parasympathetic nerves in humans. A novel approach to the cardiac autonomic nervous system. *Circulation 2001;104:2430-2435)* hat jedoch keinen Effekt auf die Sinusknotenfrequenz. Eine epikardiale oder transvaskuläre Stimulation beider Nervenplexus führt in zu einer Verkürzung der lokalen atrialen Refraktärzeit in der Umgebung des jeweiligen Nervenplexus und kann zu einer leichten Verringerung der artrialen Kontraktilität führen. Hingegen wird die ventrikuläre Pumpkraft oder Refraktärzeit nicht wesentlich beeinflusst. Die transvaskulären Nervenstimulations-Reizschwellen sind deutlich höher als die epikardialen Reizschwellen (Schauerte P et al. Ventricular Rate Control During Atrial Fibrillation by Cardiac Parasympathetic Nerve Stimulation. A Transvenous Approach. *J Am Coll Cardiol.* 1999;34:2043-2050). Parasympathische Fasern, die den Sinus- und den AV Knoten innervieren, können auch extravaskulär oder transvaskulär entlang/in der Hohlvene elektrisch stimuliert werden, was ebenfalls zu negativ chronotropen und dromotropen Wirkungen führt (Schauerte P et al. Ventricular Rate Control During Atrial Fibrillation by Cardiac Parasympathetic Nerve Stimulation. A Transvenous Approach. *J Am Coll Cardiol.* 1999;34:2043-2050; Schauerte P et al. Transvenous parasympathetic nerve stimulation in the inferior vena cava and atrioventricular conduction. *J Cardiovasc Electrophysiol.* 2000;11:64-69; Schauerte P et al. Transvenous parasympathetic cardiac nerve Stimulation: An approach for stable sinus rate control. *J Cardiovasc Electrophysiol*. 1999;10:1517-1524; Schauerte P et al. Die Behandlung des tachykarden Vorhofflimmerns durch kathetergestützte elektrische Stimulation des kardialen parasympathischen Nervensystem. *Z Kardiol.* 2000;89:766-773; Schauerte P et al. Transvascular radiofrequency current catheter ablation of parasympathetic cardiac nerves abolishes vagally mediated atrial fibrillation. *Circulation*. 2000;28:2774-2780).

Darüber hinaus kommt es hierbei zu einer Verkürzung der atrialen jedoch Verlängerung der ventrikulären Refraktärzeit sowie zu einer leichten Verminderung der atrialen Kontraktilität. Parasympathische Fasern, die den Sinusund den AV Knoten innervieren, können ebenfalls entlang der rechten oder linken Pulmonalarterie stimuliert werden, was ebenfalls negativ chronotrope und dromotrope Wirkungen auslöst. Bei den parasympathischen Fasern entlang der oberen Hohlvene sowie entlang der Pulmonalarterien handelt es sich um präganglionäre Nervenfasern während im inferioren interatrialen Plexus sowohl prä- als auch postganglionäre Nervenfasern akkumulieren (Schauerte P et al. Transvascular radiofrequency current catheter ablation of parasympathetic cardiac nerves abolishes vagally mediated atrial fibrillation. *Circulation*. 2000;28:2774-2780).

Abbildung 1 verdeutlicht den Effekt einer elektrischen Stimulation des inferioren interatrialen Plexus. Der frequenzverlangsamende Effekt ist instantan, d.h. er setzt unmittelbar mit Beginn der Nervenstimulation ein und terminiert sofort nach Beendigung der Stimulation. Darüber hinaus ist er "titrierbar, d.h. das Ausmaß der Herzfrequenzverlangsamung kann durch Wahl der entsprechenden Stimulationsspannung verändert werden.

In Abbildung 2 ist ein Beispiel der parasympathischen Stimulation des ventralen rechtsatrialen Plexus zu sehen.

Die US 6,006,134 offenbart eine gattungsgemäße Vorrichtung zum vorübergehenden Senken der Herzbewegung während einer Operation durch Neurostimulation mit einer Neurostimulationseinrichtung und einer Steuereinrichtung, die zum patientenspezifischen Beeinflussen des Betriebszustands der Neurostimulationseinrichtung ausgebildet ist. Dabei werden die Einstellungen so vorgenommen, dass durch die Neurostimulation der Herzschlag vollständig angehalten wird.

Aus der US 5,913,876 ist ebenfalls eine Vorrichtung zum vorübergehenden Senken der Herzbewegung während einer Operation durch Neurostimulation bekannt.

Bei beiden bekannten Vorrichtungen ergibt sich das Problem, dass mit zunehmender Dauer des Herzstillstandes oder der Herzfrequenzverlangsamung das Herzzeitvolumen unter einen kritischen Wert sinkt, sodass die Nervenstimulation auch stets nur für einige Sekunden eingesetzt werden kann. Daher wird die Nervenstimulation in der Regel immer nur solange aufrechterhalten, als es notwendig ist, einen Nahtstich oder wenige Nahtstiche durchzuführen. Anschließend wird für eine bestimmte Dauer die Herztätigkeit mit der sofort wieder einsetzenden natürlichen Herzfrequenz zugelassen, um die ausreichende Perfusion der Organe sicherzustellen. Hierbei ist es von Nachteil, dass durch die hohen natürlichen Wandexkursionen noch nicht vollständig vollendete Nähte hohen mechanischen Beanspruchungen ausgesetzt sind und gegebenenfalls später nochmals nachgearbeitet werden müssen, wodurch sich die Operationsdauer verlängert.

Der vorliegenden Erfindung liegt daher die Aufgabe zu Grunde, eine gattungsgemäße Vorrichtung zum vorübergehenden Senken der Herzbewegung während einer Operation zur Verfügung zu stellen, welche diesem Nachteil zumindest teilweise abhilft und insbesondere eine längere andauernde Erzielung einer ausreichenden Immobilisierung des Herzens zu einem gewissen Grad ermöglicht.

Insbesondere soll eine Vorrichtung geschaffen werden, die durch transiente intraoperative epikardiale oder transvaskuläre elektrische parasympathische Stimulation die Herzfrequenz vorübergehend senkt bzw. den Herzschlag anhält, um durch diese vorübergehende elektrische Stillegung des Herzens dem Operateur/Roboter die Führung der Operationsinstrumente am Herzen zu erleichtern.

Die Erfindung löst diese Aufgabe durch eine Vorrichtung zum vorübergehenden Senken der Herzbewegung während einer Operation, insbesondere während einer Herzoperation, mit einer Neurostimulationseinrichtung zur Stimulation die Herzfrequenz verlangsamender Nerven, die wenigstens eine Elektrodeneinrichtung mit wenigstens einer Stimulationselektrode umfasst. Erfindungsgemäß ist dabei eine mit der Neurostimulationseinrichtung verbundene Steuereinrichtung vorgesehen, die eine erste Vorgabeeinrichtung zur im Betrieb variablen Vorgabe eines elektrischen Immobilisierungsgrades des Herzens aufweist und zum patientenspezifischen Beeinflussen des Betriebszustandes der Neurostimulationseinrichtung in Abhängigkeit von dem vorgegebenen elektrischen Immobilisierungsgrad ausgebildet ist.

Jede Bradykardie geht normalerweise mit einer Erhöhung des Schlagvolumens einher. Daher würde eine intraoperative Bradykardie zwar die Zahl der Kontraktionen pro Minute reduzieren, eine Einzelkontraktion würde jedoch zu einer größeren Wandeinwärts-Wandauswärtsbewegung des Herzens führen, was einer Immobilisation des Herzens entgegenwirken würde. Mit anderen Worten, häufige leichte Wandexkursionen ohne Nervenstimulation würden durch wenige große Wandbewegungen während einer Nervenstimulation ersetzt.

Um diesen Nachteil einer Bradykardisierung auszugleichen, sieht die Erfindung in einer vorteilhaften Modifikation die Kombination des Neurostimulators mit einer weiteren mit der Steuereinrichtung verbundenen Bewegungsreduktionseinrichtung in Form einer intravaskulären/intrakardialen Pumpe vor, die einen Teil der mechanischen Pumpfunktion des Herzens übernimmt. Das von der Pumpe übernommene Herzzeitvolumen kann jederzeit an das Ausmaß der Bradykardie angepasst werden, d.h. je größer die Bradykardie ist, desto größer ist der Anteil des Herzzeitvolumens, das durch die Pumpe transportiert wird. Auf diese Weise wird ein ausreichendes Herzzeitvolumen während der Bradykardie aufrechterhalten, zugleich aber auch das vermehrte Schlagvolumen und die konsekutiv vermehrte Wandexkursion einer Bradykardie verringert, was zu einer effektiveren Immobilisation des Herzens führt als eine intravaskuläre Pumpe oder Nervenstimulation allein.

Auch die Kombination einer Neurostimulationseinheit mit einer Bewegungsreduktionseinrichtung in Form einer Stabilisationseinrichtung zur Stabilisierung der Herzwand, beispielsweise einer lokalen Stabilisations/Immobilisationsvorrichtung, ist gemäß der vorliegenden Erfindung vorgesehen. Hierbei soll die durch die Neurostimulationseinheit erzielte Bradykardie und elektrische Immobilisation additiv zur lokalen Immobilisation durch die lokalen Stabilisationssysteme wirken.

Weitere bevorzugte Ausführungsformen der Erfindung ergeben sich aus den Unteransprüchen bzw. der nachstehenden Beschreibung bevorzugte Ausführungsbeispiele, welche auf die beigefügten Zeichnungen Bezug nimmt. Es zeigt:
- Figur 1: ein Elektrokardiogramm bei einer elektrischen Stimulation des inferioren interatrialen Plexus;

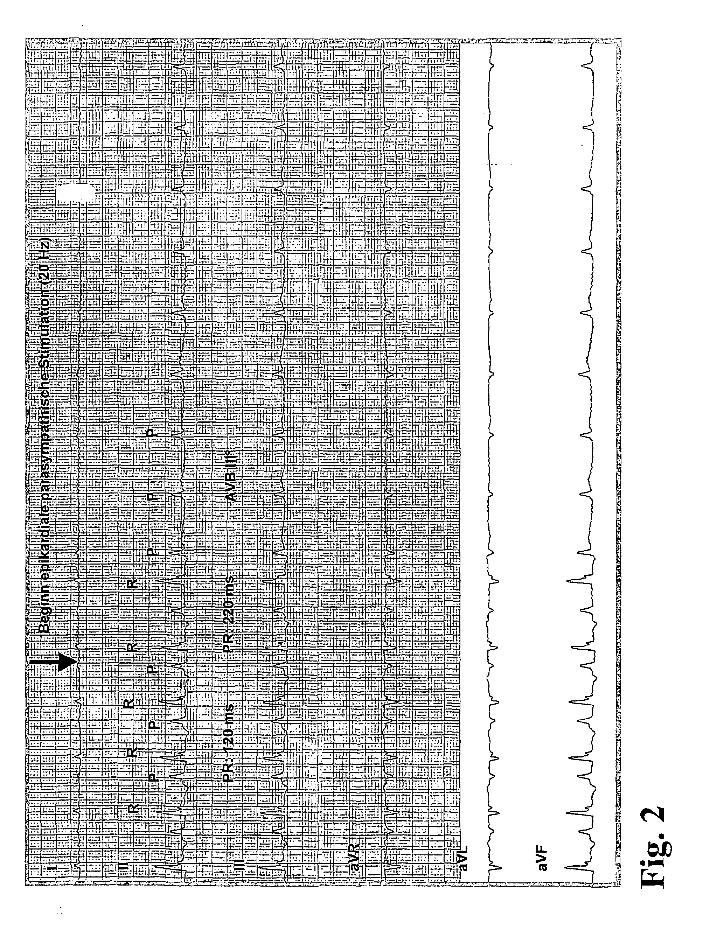

- Figur 2: ein Elektrokardiogramm bei parasympathischer Stimulation des ventralen rechtsatrialen Plexus;

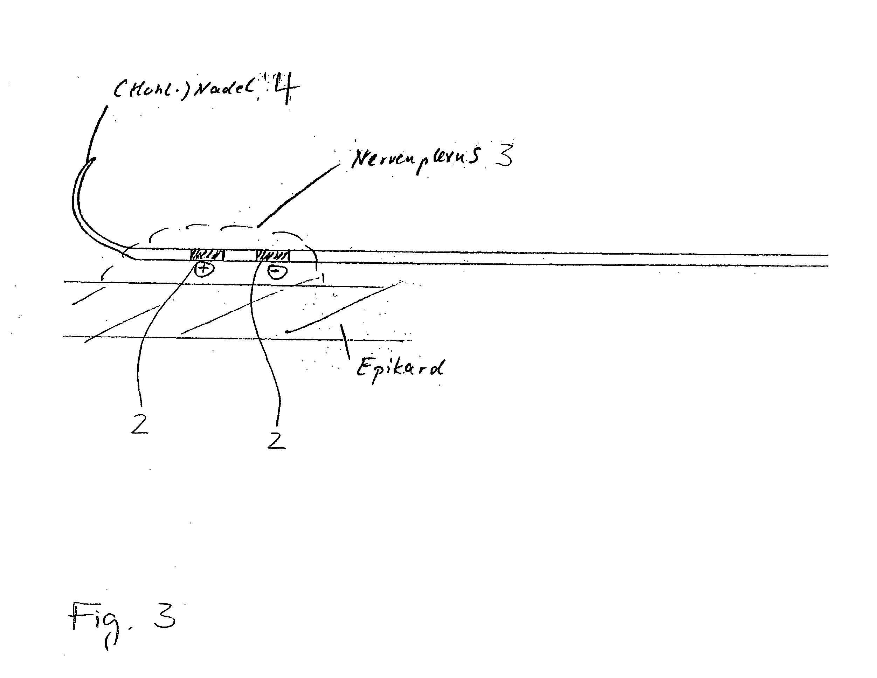

- Figur 3: ein bevorzugtes Ausführungsbeispiel einer Elektrodeneinrichtung einer erfindungsgemäßen Vorrichtung;

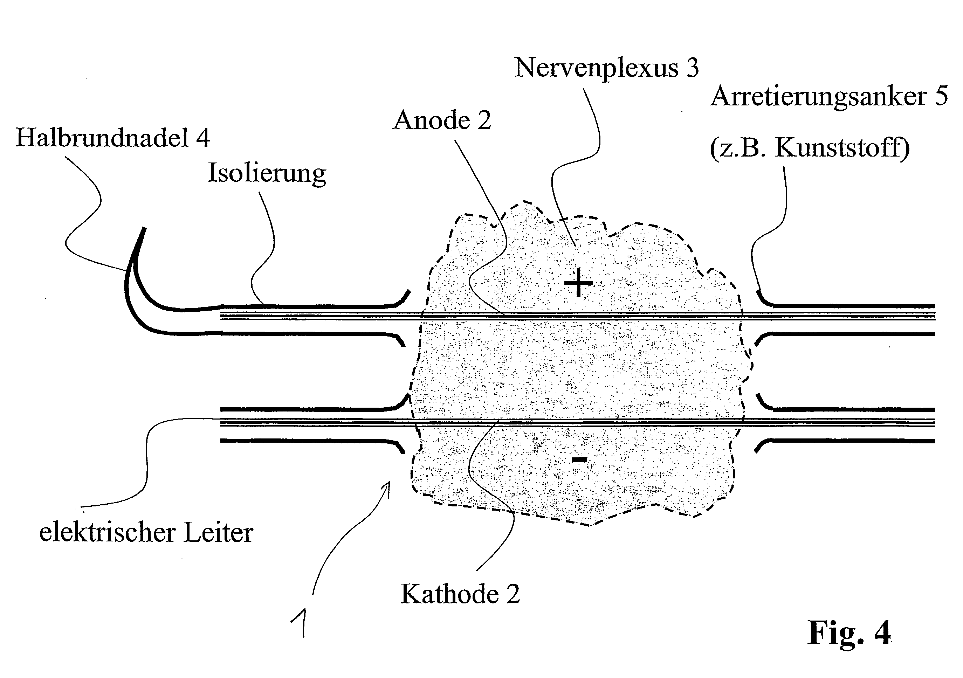

- Figur 4: ein weiteres bevorzugtes Ausführungsbeispiel einer Elektrodeneinrichtung einer erfindungsgemäßen Vorrichtung;

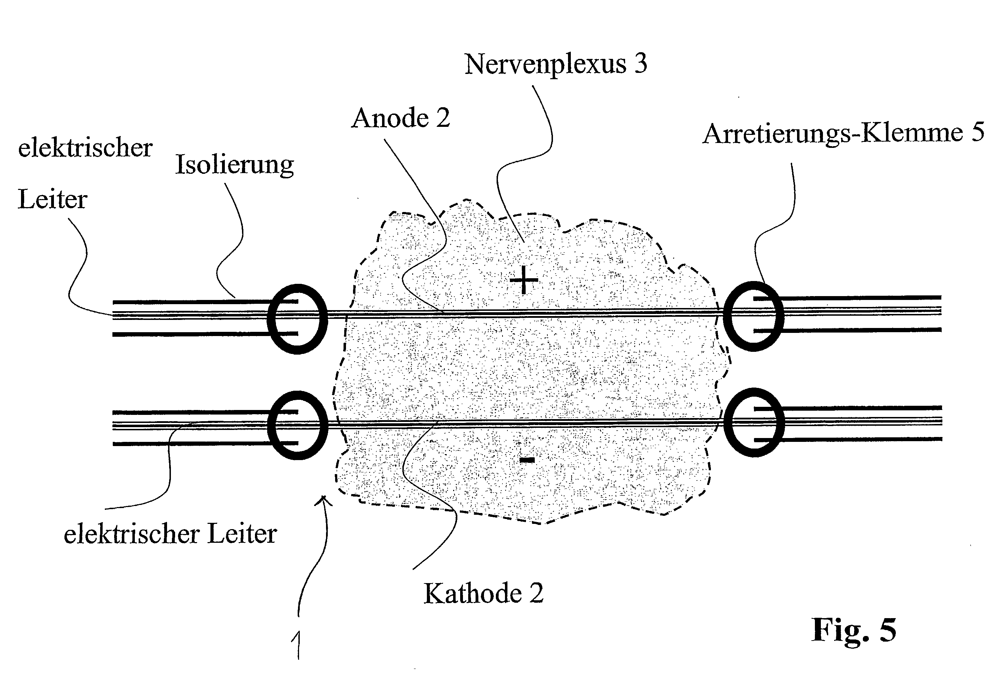

- Figur 5: ein weiteres bevorzugtes Ausführungsbeispiel einer Elektrodeneinrichtung einer erfindungsgemäßen Vorrichtung;

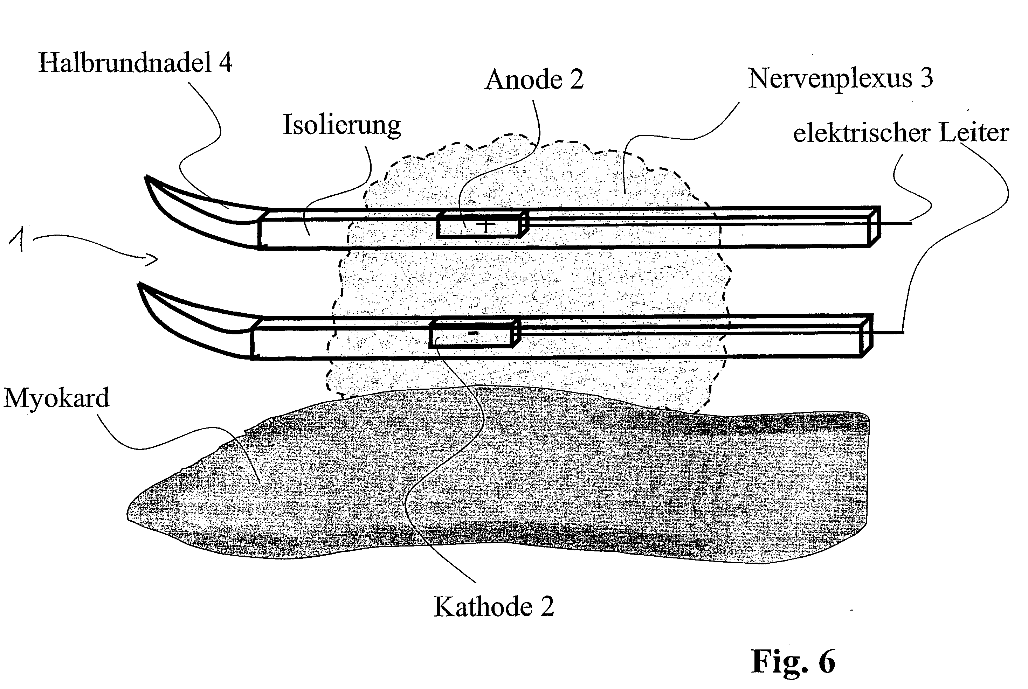

- Figur 6: ein weiteres bevorzugtes Ausführungsbeispiel einer Elektrodeneinrichtung einer erfindungsgemäßen Vorrichtung;

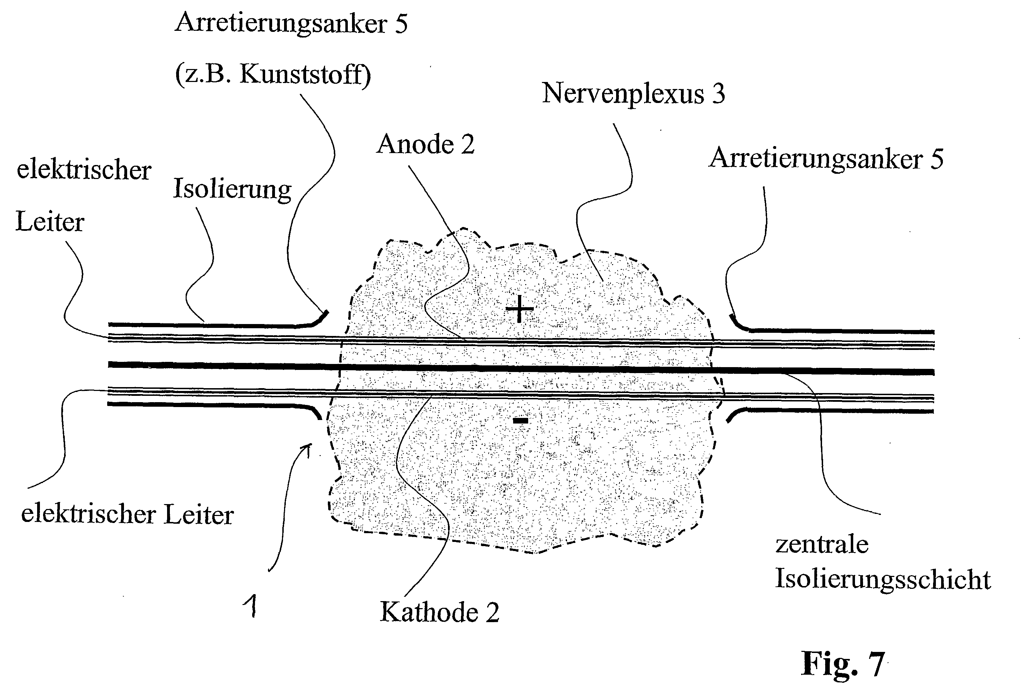

- Figur 7: ein weiteres bevorzugtes Ausführungsbeispiel einer Elektrodeneinrichtung einer erfindungsgemäßen Vorrichtung;

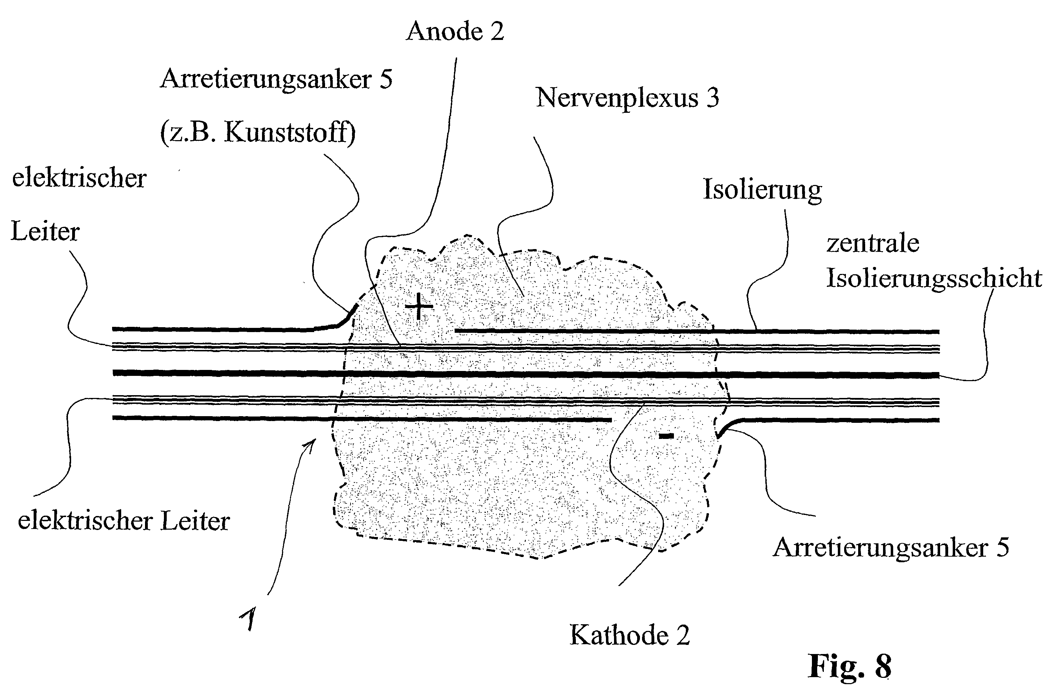

- Figur 8: ein weiteres bevorzugtes Ausführungsbeispiel einer Elektrodeneinrichtung einer erfindungsgemäßen Vorrichtung;

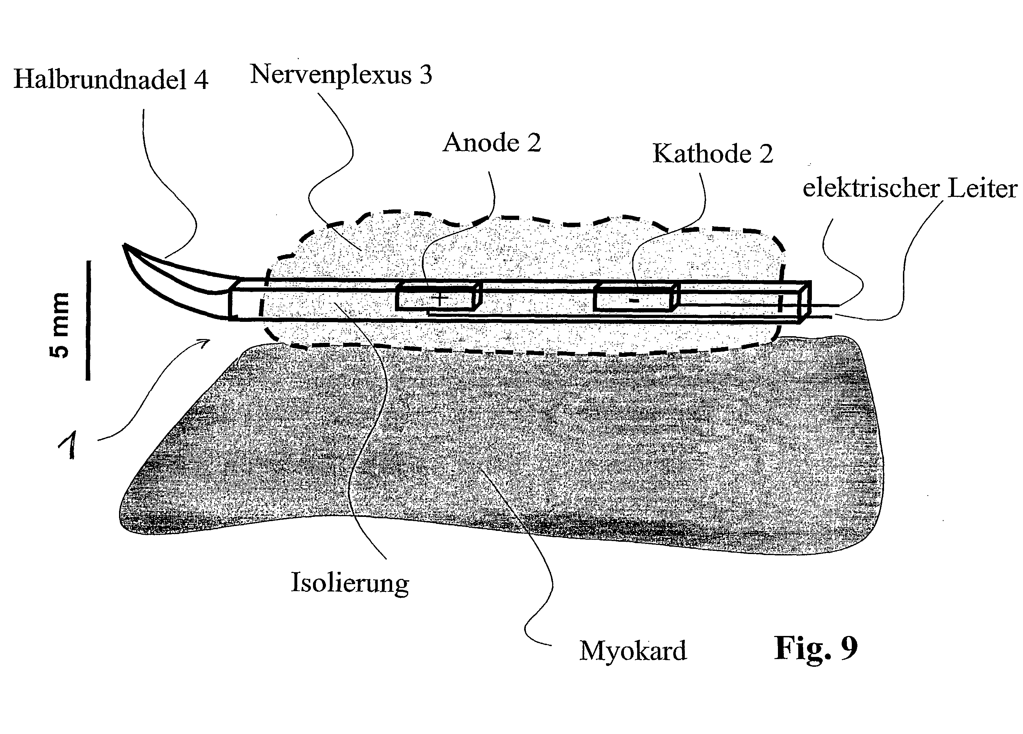

- Figur 9: ein weiteres bevorzugtes Ausführungsbeispiel einer Elektrodeneinrichtung einer erfindungsgemäßen Vorrichtung;

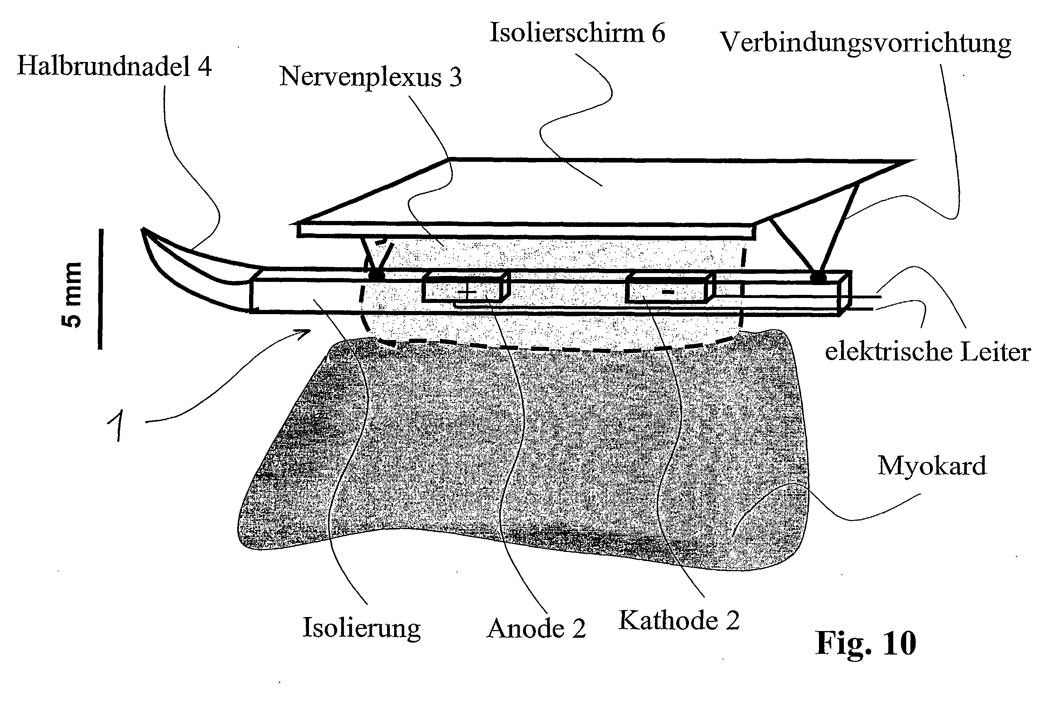

- Figur 10: ein weiteres bevorzugtes Ausführungsbeispiel einer Elektrodeneinrichtung einer erfindungsgemäßen Vorrichtung;

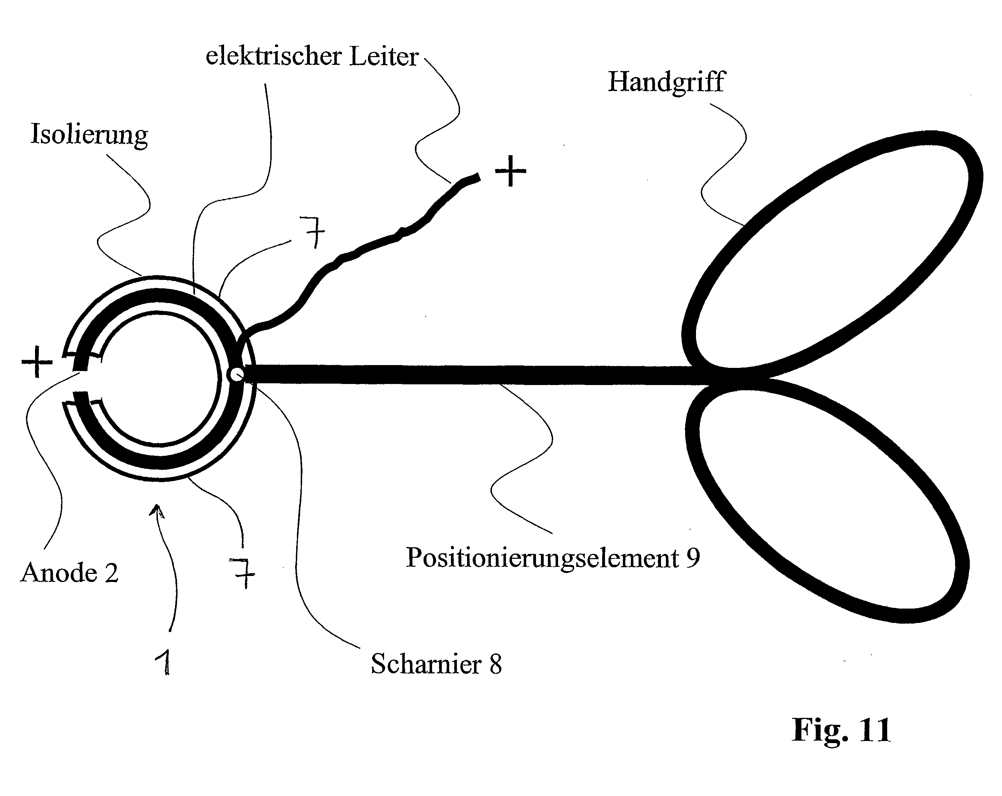

- Figur 11: ein weiteres bevorzugtes Ausführungsbeispiel einer Elektrodeneinrichtung einer erfindungsgemäßen Vorrichtung;

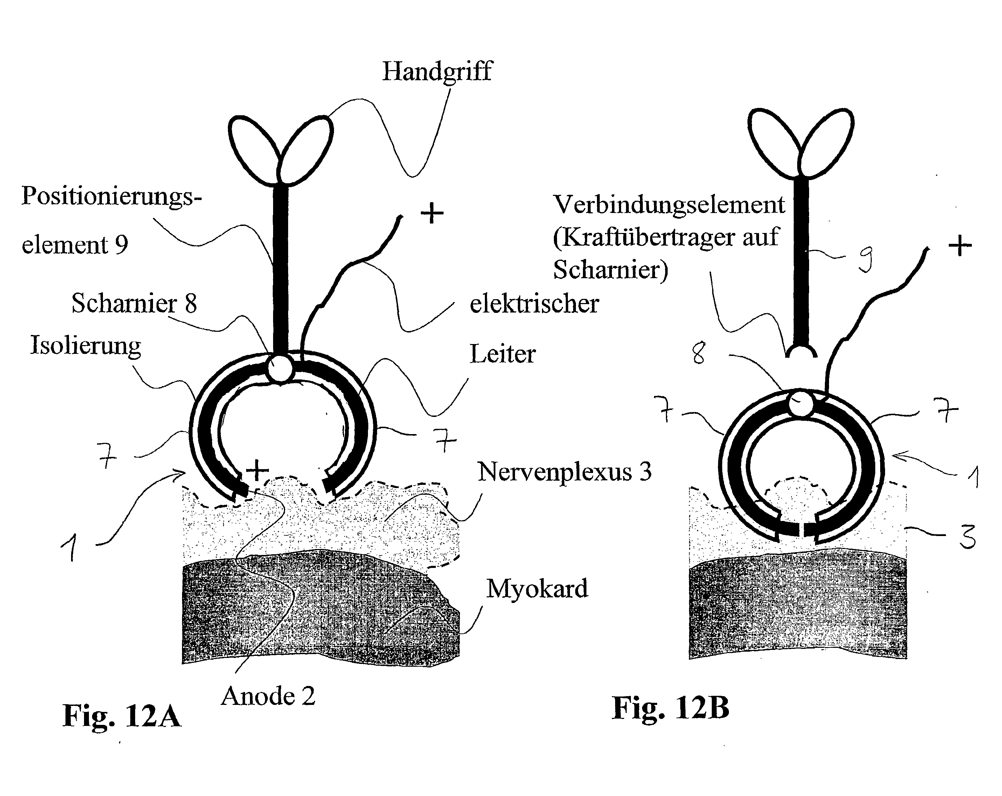

- Figur 12: ein weiteres bevorzugtes Ausführungsbeispiel einer Elektrodeneinrichtung einer erfindungsgemäßen Vorrichtung;

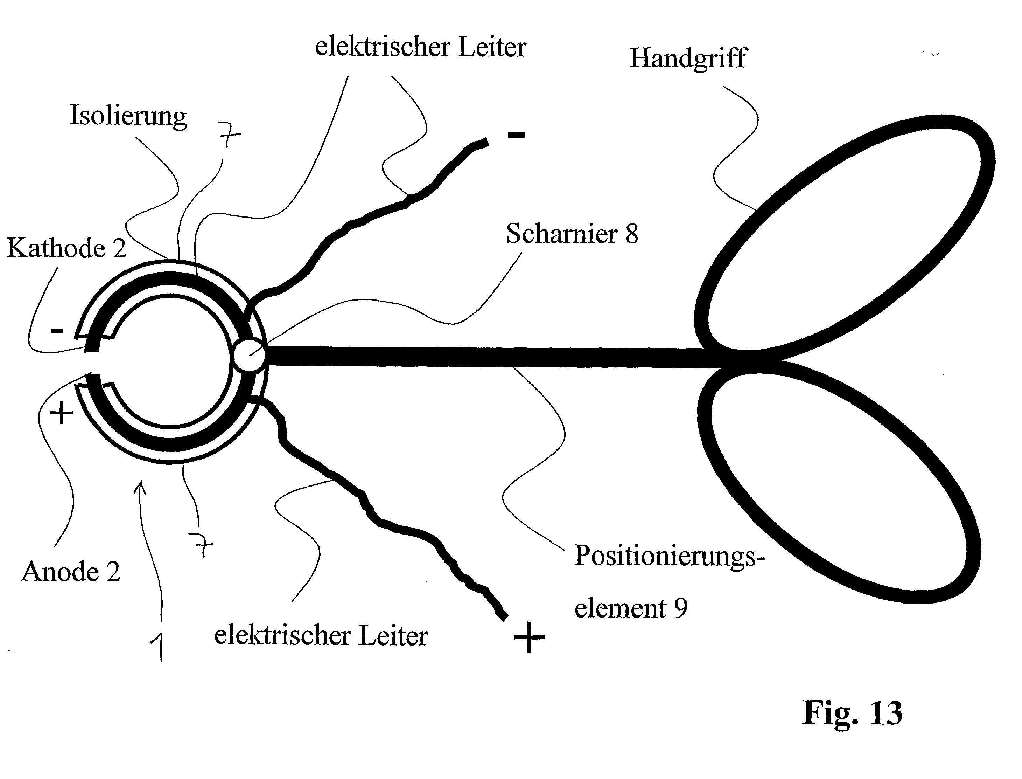

- Figur 13: ein weiteres bevorzugtes Ausführungsbeispiel einer Elektrodeneinrichtung einer erfindungsgemäßen Vorrichtung;

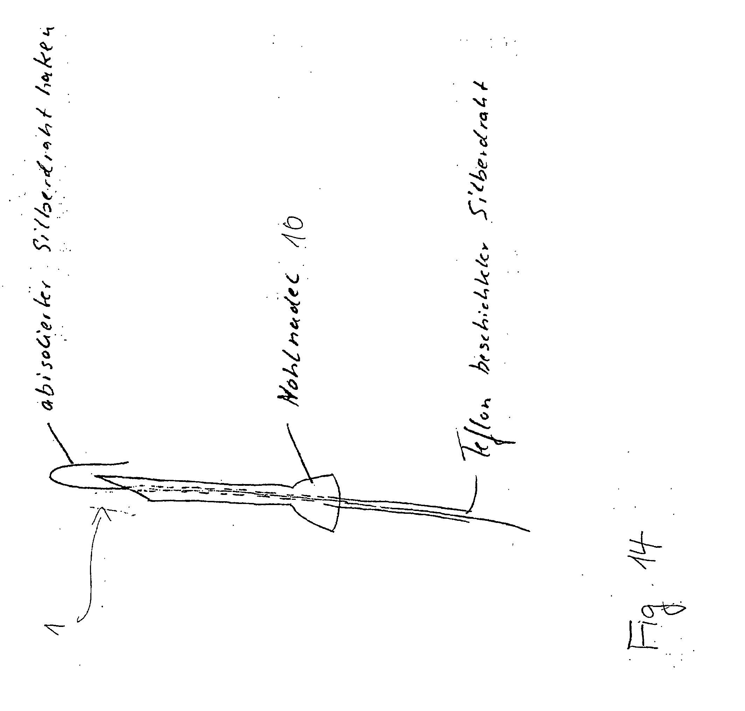

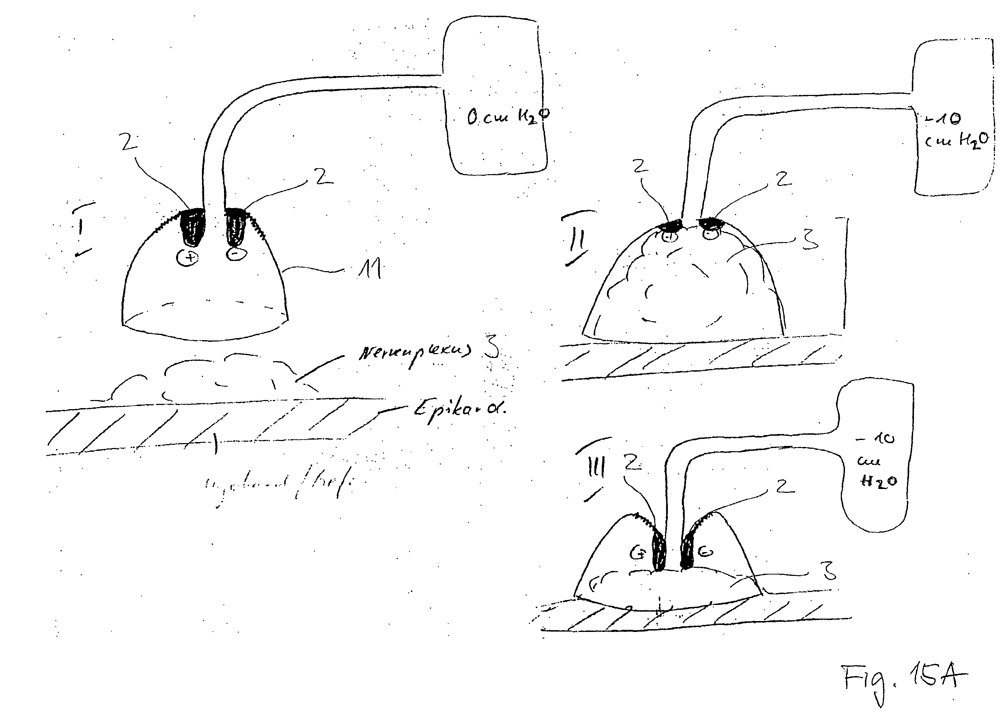

- Figur 14: ein weiteres bevorzugtes Ausführungsbeispiel einer Elektrodeneinrichtung einer erfindungsgemäßen Vorrichtung;

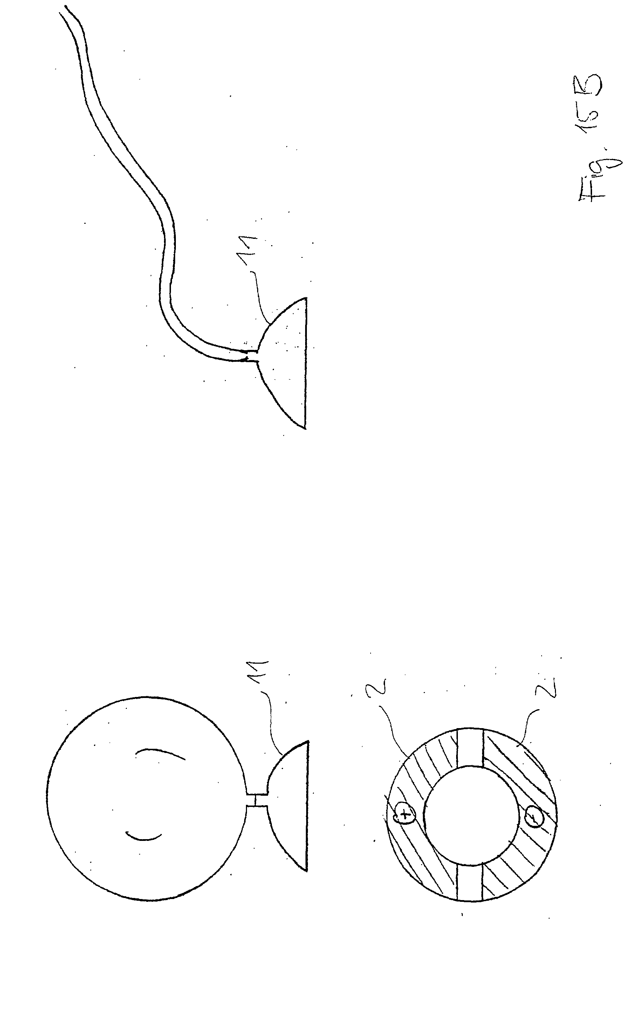

- Figur 15A: und 15B ein weiteres bevorzugtes Ausführungsbeispiel einer Elektrodeneinrichtung einer erfindungsgemäßen Vorrichtung;

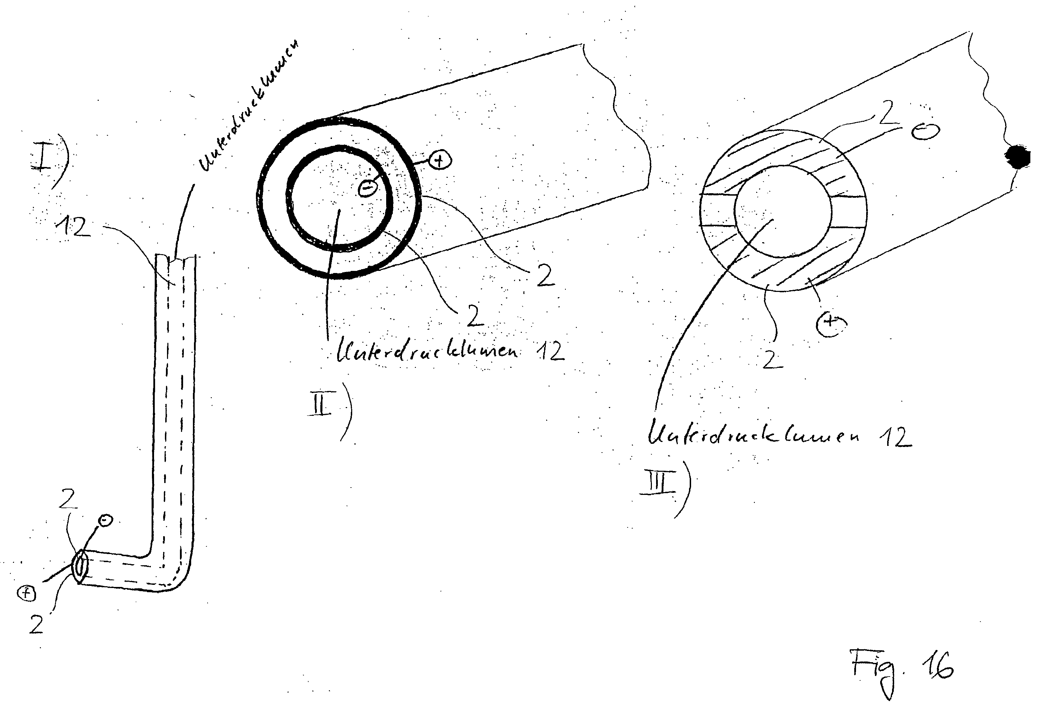

- Figur 16: ein weiteres bevorzugtes Ausführungsbeispiel einer Elektrodeneinrichtung einer erfindungsgemäßen Vorrichtung;

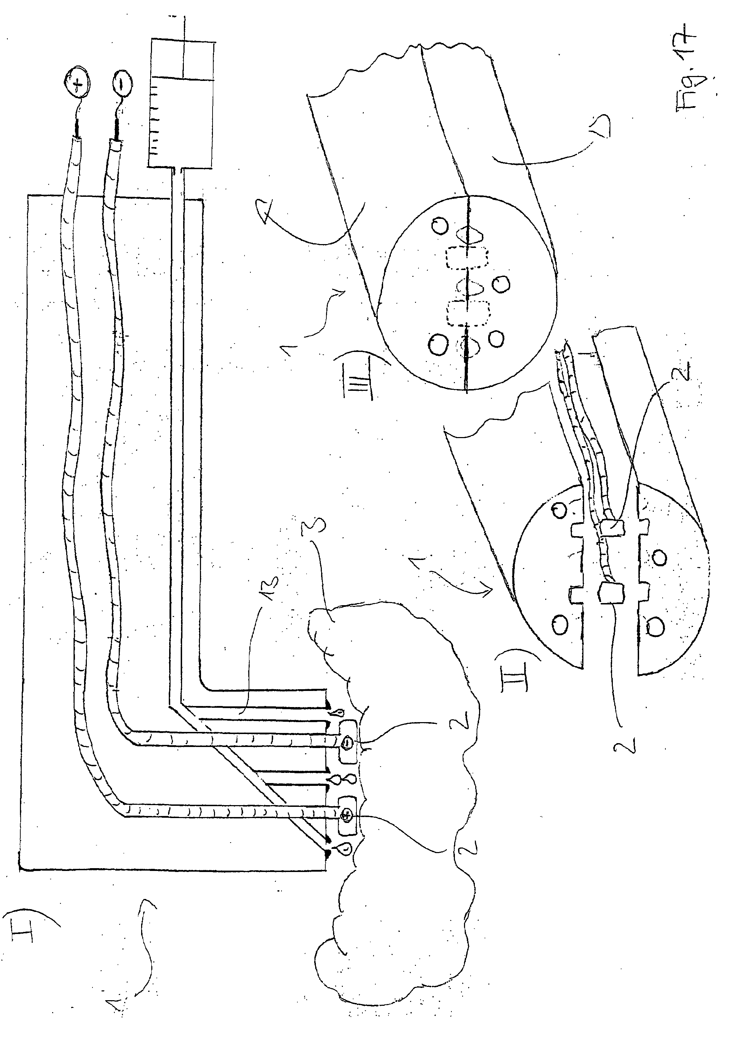

- Figur 17: ein weiteres bevorzugtes Ausführungsbeispiel einer Elektrodeneinrichtung einer erfindungsgemäßen Vorrichtung;

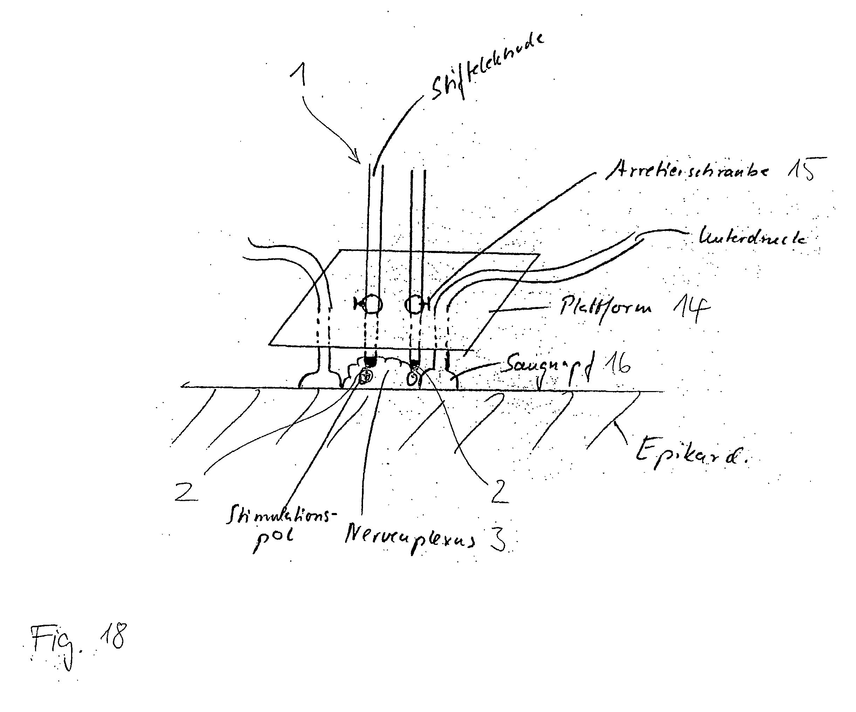

- Figur 18: ein weiteres bevorzugtes Ausführungsbeispiel einer Elektrodeneinrichtung einer erfindungsgemäßen Vorrichtung;

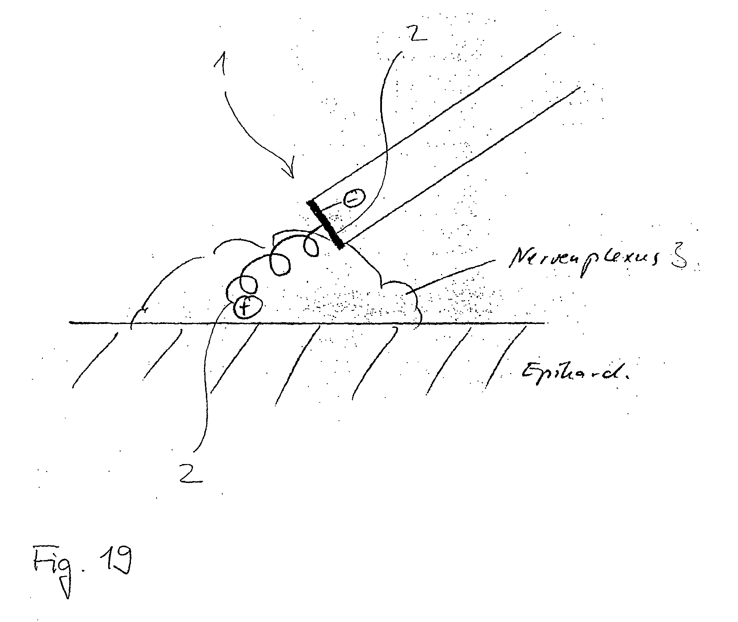

- Figur 19: ein weiteres bevorzugtes Ausführungsbeispiel einer Elektrodeneinrichtung einer erfindungsgemäßen Vorrichtung;

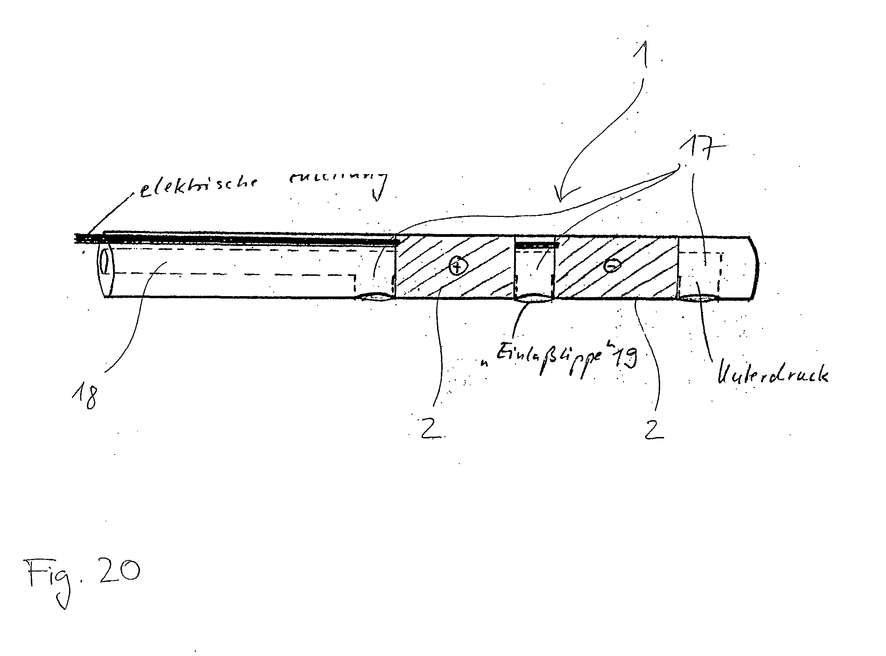

- Figur 20: ein weiteres bevorzugtes Ausführungsbeispiel einer Elektrodeneinrichtung einer erfindungsgemäßen Vorrichtung;

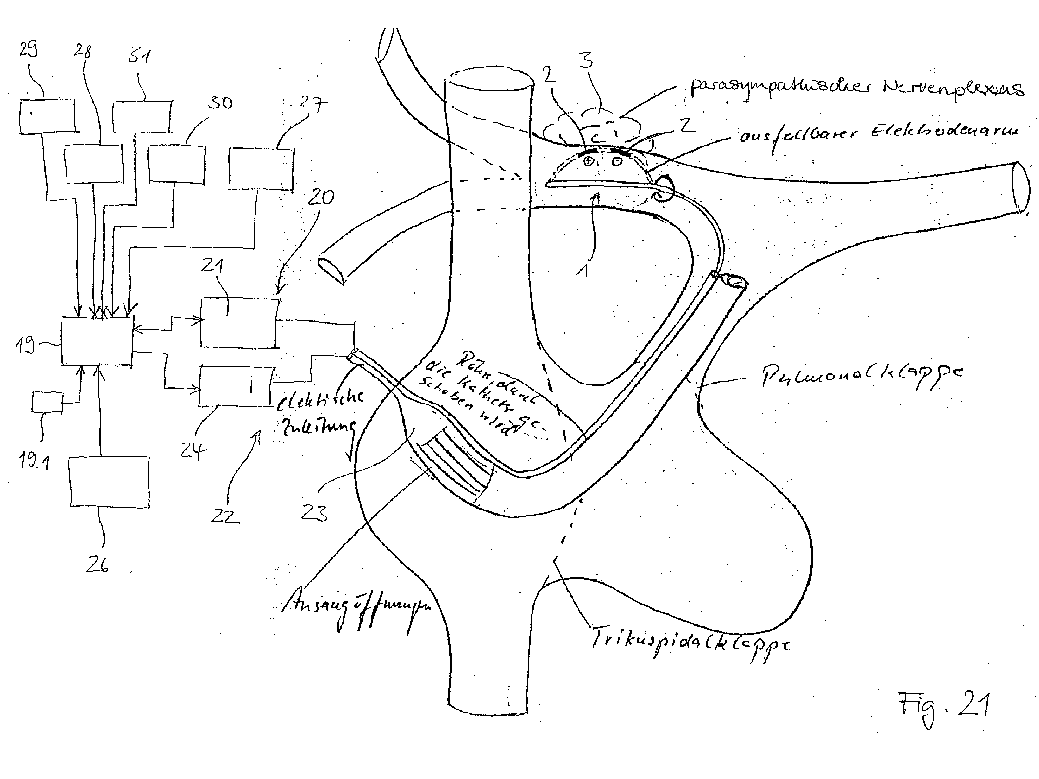

- Figur 21: ein weiteres bevorzugtes Ausführungsbeispiel einer erfindungsgemäßen Vorrichtung;

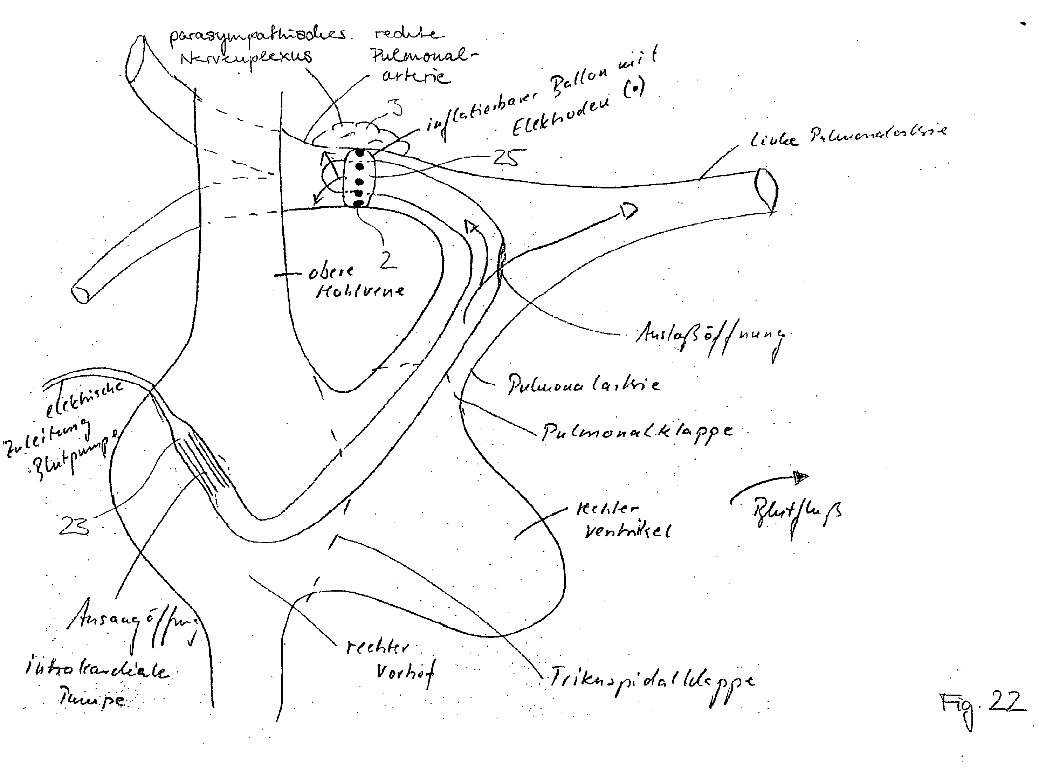

- Figur 22: ein weiteres bevorzugtes Ausführungsbeispiel einer erfindungsgemäßen Vorrichtung;

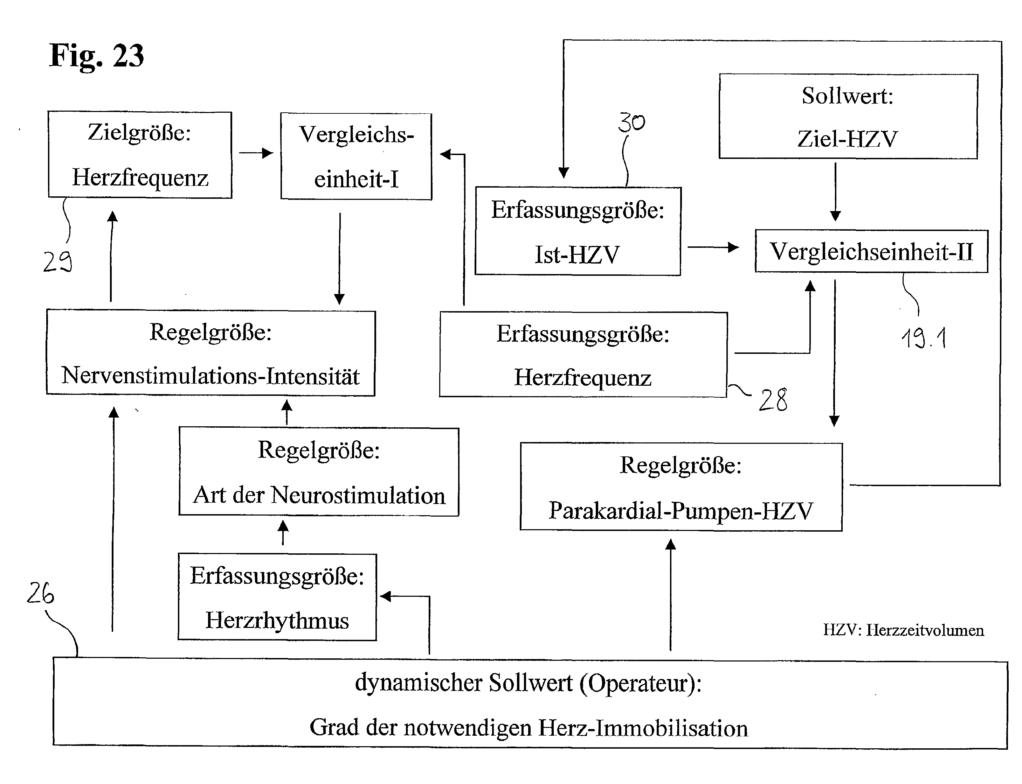

- Figur 23: ein Blockschaltbild des Regelkreises gemäß einer weiteren bevorzugten Ausführung der erfindungsgemäßen Vorrichtung;

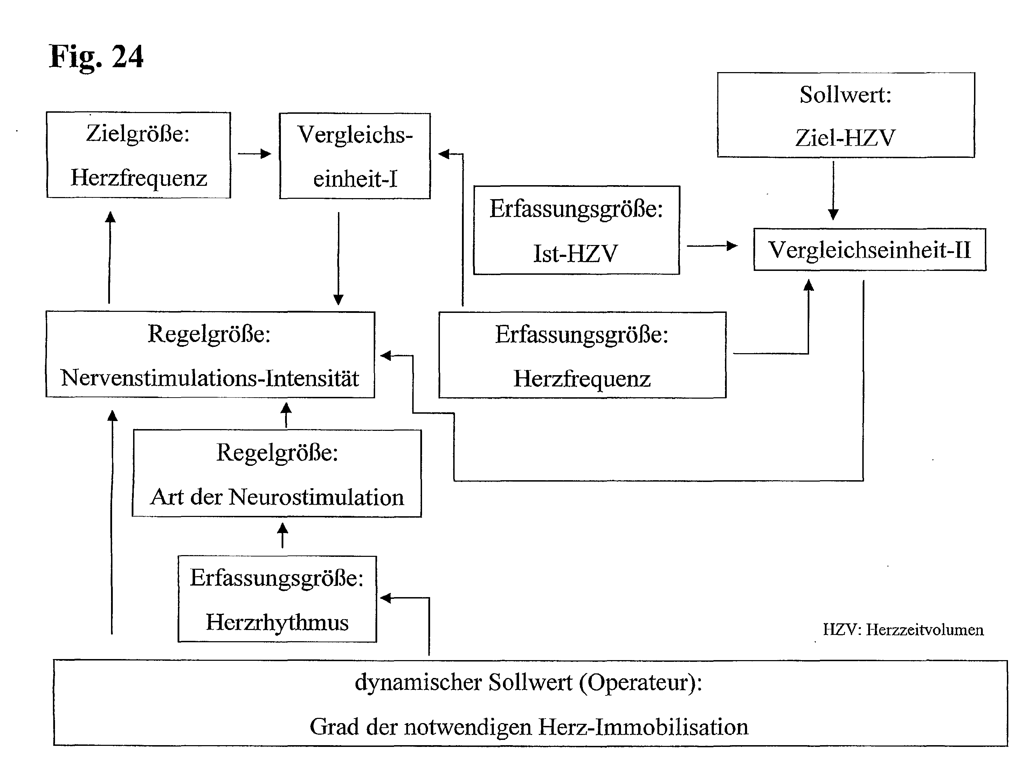

- Figur 24: ein Blockschaltbild des Regelkreises gemäß einer weiteren bevorzugten Ausführung der erfindungsgemäßen Vorrichtung.

Figur 1 zeigt ein Beispiel der parasympathischen Stimulation des ventralen rechtsatrialen Plexus mit konsekutiver Sinusbradykardie (P-P Intervall 440 ms). Zur Vermeidung einer atrialen myokardialen Stimulation wurden die hochfrequenten (200 Hz) Nervenstimuli (*) in der atrialen Refraktärzeit abgegeben. Unmittelbar nach Ende der Nervenstimulation (dicker vertikaler Pfeil) in der atrialen Refraktärzeit kommt es wieder zum Anstieg der Sinusknotenfrequenz (P-P Intervall 300 ms). Mit R ist dabei die R-Zacke, mit P die P-Welle bezeichnet.

Figur 2 zeigt den Effekt einer epikardialen elektrischen Stimulation des inferioren rechtsatrialen Plexus am Hund. Der frequenzverlangsamende Effekt ist instantan, d.h. er setzt unmittelbar mit Beginn der Nervenstimulation ein und terminiert sofort nach Beendigung der Stimulation. Auch hier bezeichnet R die R-Zacke und P die P-Welle.

Gemäß der Erfindung umfasst die Neurostimulationseinrichtung eine Elektrodeneinrichtung in Form einer Stimulationselektrode 1, die epikardial auf dem ventralen rechtsatrialen Plexus, dem inferioren interatrialen Plexus, der oberen Hohlvene oder der rechten bzw. linken Pulmonalarterie fixiert wird. Das Vorbringen einer derartigen Elektrode kann nach Durchführen einer Thorakotomie im offenen Thorax erfolgen. Alternativ können die Nervenstimulationselektroden jedoch auch durch Trokare endoskopisch/robotergestützt an den Stimulationsorten platziert werden. Die Elektrode weist in typischer Ausführung ein bis zwei elektrisch leitende Stimulationspole 2.1 auf, deren wirksame Stimulationsfläche zwischen 1 und 100 mm² (bevorzugte Ausführung: 4-9 mm²) beträgt **(vgl. Figuren 3-7).** Die Stimulationspole 2 können Teil eines mit Kunststoff isolierten elektrischen Leiters (Stimulationsdraht) sein, wobei die Isolierung im Bereich des Stimulationspols 2 entfernt ist. Der Stimulationsdraht wird durch den epikardialen Nervenplexus 3 gezogen, sodass der Stimulationspol 2 im Nervenplexus zu liegen kommt. Zur Erleichterung des Einführens in den Nervenplexus 3 weist eine Ausführungsform des Stimulationsdrahtes an der Spitze eine Verjüngung oder Nadel 4 auf, die ein Durchstechen des Nervenplexus 3 ermöglicht. In typischer Ausführung handelt es sich hierbei um eine (Halb-) Rundnadel 4, die es ermöglicht, den epikardialen Nervenplexus 3 oberflächlich zu durchstechen. Um eine bipolare Stimulation des Nervenplexus zu ermöglichen, werden zwei Stimulationsdrähte in einem Abstand von ca. 2-10 mm im Nervenplexus 3 platziert. Alternativ können zwei jeweils gegeneinander isolierte elektrische Leiter in einer gemeinsamen Stimulationselektrode vereint sein **(vgl. Figuren 6-8).** Die zwei isolierten Leiter weisen in verschiedenen Abständen hinter der Elektrodenspitze 4 jeweils einen Stimulationspol 2 auf.

Um eine Dislokation der Stimulationselektrode 1 aus dem Nervenplexus 3 zu verhindern, befindet sich in typischer Ausführung an beiden Seiten der Stimulationselektrode 1 eine Arretiervorrichtung 5. Hierbei kann es sich beispielhaft um zwei Kunststoffanker an beiden Seiten der Stimulationselektrode 1 handeln (vgl. Figuren 3,6,7) oder um eine nach Platzierung der Elektrode 1 zu beiden Seiten angebrachte Klemme **(vgl. Figur 4).**

Um v.a. bei Stimulation des inferioren interatrialen Plexus 3 eine myokardiale Stimulation des angrenzenden oder überliegenden Ventrikelmyokards zu vermeiden, kann bei einer besonderen Ausführungsform eine Abschirmeinrichtung in Form einer Isolierkappe 6, die aus elektrisch nicht leitfähigem Material besteht, zu beiden Seiten der Stimulationselektroden 1 mit einer Arretiervorrichtung 5 an der Stimulationselektrode vorübergehend befestigt werden, sodass die im Plexus 3 liegenden Stimulationspole 2 und der Plexus 3 vom umgebenden/überliegenden Myokard isoliert sind **(vgl. Figur 9).** Alternativ können die Stimulationspole 2, die auf einer flächenhaft ausgebildeten Stimulationselektrode 1 angebracht sind, auf einer Seite elektrisch abgeschirmt **(vgl. Figur 8)** sein. Dies ermöglicht, die Stimulationselektrode 1 mit der elektrisch leitenden Fläche/Seite zum Epikard gerichtet zu platzieren und eine Abschirmung 6 gegenüber dem überliegenden (ventrikulären) Myokard zu erzielen. Ebenso kann die Stimulationselektrode 1 mit der elektrisch leitenden Fläche/Seite aber auch dem Epikard abgewandt platziert werden, sodass die isolierte Fläche 6 dem Epikard anliegt. Hierdurch kommt die Elektrode 1 zwischen dem Epikard und dem überliegenden Plexus 3 zu liegen, sodass eine gleichzeitige (atriale) myokardiale Stimulation während Nervenstimulation verhindert wird. Bei einer weiteren Ausführungsform kann eine flächenhaft ausgebildete Stimulationselektrode 1 auch mit einer Isolierkappe 6 kombiniert werden. In diesem Fall wird die elektrisch leitenden Fläche der Elektrode 1 im Plexus 3 jedoch dem Epikard abgewandt plaziert und die Isolierkappe 6 zu beiden Seiten der Stimulationselektroden mit einer Arretiervorrichtung an der Stimulationselektrode befestigt **(vgl. Figur 9).** Dies kann eine elektrische Stimulation des unter dem Plexus 3 liegenden (atrialen) Myokards wie auch des überliegenden (ventrikulären) Myokards verhindern.

Bei einer anderen Ausführungsform besteht die Stimulationselektrode aus einer Ringelektrode 1, die sich aus zwei halbrunden Armen 7 zusammensetzt **(Abbildung 10-12).** Die proximalen Enden sind in einem Scharnier 8 beweglich fixiert, die distalen Enden berühren sich Ende zu Ende oder überlappen an den Enden. Die distalen Enden beider Halbkreise können durch einen Zug-/Druckmechanismus, der am Scharnier 8 der Halbkreise ansetzt und der durch ein Positionierungselement 9 übertragen wird, auseinandergezogen werden und verhalten sich so wie zwei Zangenarme 7, mit denen das Gewebe des Nervenplexus 3 ergriffen werden kann **(vgl. Figur 12 A).** Aufgrund der elastischen Rückstellkräfte der beiden Halbkreise oder durch eine erneute Krafteinwirkung am Scharnier 8 schließen sich die Arme 7 des Kreises und sind so im Nervenplexus 3 fixiert. Die Zangenarmen 7 wirken daher wie ein Befestigungselement zum Festklemmen der Ringelektrode 1. Das Positionierungselement 9 wird nun vom Scharnier 8 entfernt **(vgl. Figur 12 B).** Beide Halbkreise 7 sind aus elektrisch leitfähigem Material beschaffen und bis auf die distalen Enden mit einer nicht elektrisch leitenden Substanz überzogen. Die Halbkreise 7 sind mit einem flexiblen, nach außen elektrisch isolierten elektrischen Leiter verbunden. Durch Platzierung zweier derartiger Ringelektroden 1 in einem Nervenplexus 3 gelingt die bipolare Stimulation. Eine Variante dieser Ausführungsform sieht die Anordnung von zwei gegensinnigen elektrischen Polen 2 auf einer Ringelektrode 1 vor **(vgl. Figur 13).**

Eine alternative Ausführungsform einer Stimulationselektrode 1 besteht aus einem dünnen flexiblen Silberdraht, der z.B. mit Teflon beschichtet ist, wobei die Isolierung an der Spitze des Silberdrahtes auf eine Länge von ca. 5-10 mm entfernt ist. Dieser Draht kann durch eine herkömmliche Hohlnadel 10 aus Stahl, wie sie zur venösen Gefäßpunktion benutzt wird, eingeführt werden (z.B. 20 Gauge Nadel) **(Abbildung 14).** Sobald der Draht an der Spitze aus der Hohlnadel ca. 5 mm austritt, wird die Drahtspitze an ihrem Austritt aus der Hohlnadel zu einem Haken umgebogen. Die Hohlnadel 10, die auch als Rundnadel ausgebildet sein kann, wird dann in den Nervenplexus 3 ("fat pad") eingestochen, sodass der nicht isolierte Drahthaken im Nervenplexus zu liegen kommt. Alsdann wird die Nadel 10 vorsichtig zurückgezogen, sodass der Drahthaken im Nervenplexus hängen bleibt. Zur bipolaren elektrischen Stimulation des Nervenplexus werden zwei dieser Teflon beschichteten Stimulationsdrähte 1 mit den Stimulationspolen 2 in jeweils einen Plexus 3 platziert. Der Abstand der beiden Stimulationspole 2 soll zwischen 1 und 10 mm betragen.

Bei einer modifizierten Ausführungsform sind die Stimulationselektroden in einer Ansaugeinrichtungen in Form einer Saugglocke oder Saugröhre 11 inkorporiert, an der ein permanenter Unterdruck angelegt wird, um die Stimulationspole 2 epikardial auf den Nervenplexus 3 oder extravaskulär auf Gefäßen sicher zu fixieren (vgl. Figur **15**). Die Saugglocke 11 hat die Form einer Halbkugel. Der größte Durchmesser der Halbkugel beträgt 5 -15 mm mit einem typischen Diameter von 5 mm. Bei einer bevorzugten Variante besteht die Saugglocke 11 aus Kunststoff. An der Saugglocke 11 ist eine Einlassöffnung angebracht, die mit einem Saugschlauch verbundenen ist, über den ein Unterdruck angelegt werden kann. Der Unterdruck kann durch einen externen Sog über einen Schlauch appliziert werden oder durch ein lokales Unterdruck-Reservoir abgelegt werden. Ein derartiges lokales Unterdruck-Reservoir kann z.B. ein kleiner Gummiball sein, der mit einem Auslassventil versehen ist und der mit der Einlassöffnung der Saugglocke 11 verbunden ist. Durch manuelle Kompression des Gummiballs entweicht bei gleichzeitigem Aufsetzten der Saugglocke 11 auf den Nervenplexus 3 bzw. Gefäß Luft über das Auslassventil. Nach Aufheben der Kompression kommt es durch die elastischen Rückstellkräfte des Ballons zu einem Unterdruck, der den Nervenplexus 3 in die Saugglocke 11 zieht und der so zur Fixierung der Saugglocke 11 und Stimulationspole 2 auf dem Nervenplexus 3 bzw. Gefäß führt.

An der Innenseite der Saugglocke befinden sich neben der Einlassöffnung zwei metallene Stimulationspole 2, die mit dünnen elektrischen Leitern, die entlang des Saugschlauches fixiert sind, verbunden sind. Die Saugglocke 11 wird unter Anlage eines Unterdrucks auf den ventralen/inferioren interatrialen Plexus/ obere Hohlvene/rechte oder linke Pulmonalarterie aufgesetzt. Durch den Unterdruck wird das Fett- und Nervengewebe in die Halbkugel gesaugt, sodass es in Kontakt mit den Stimulationspolen 2 kommt. Um eine Dislokation der Saugelektrode z.B. bei Luxation des Herzens aus dem Herzbeutel zu vermeiden, kann der Unterdruck kurzfristig erhöht werden. Eine alternative Ausführungsform sieht zwei Stimulationselektroden an der Auflagefläche der Saugglocke 11 vor, die im Bereich der Zirkumferenz der Saugglocke 11 Kontakt mit dem epikardialen Nervenplexus 3 haben. Bei beiden Ausführungsformen kann die Auflagefläche eben, konkav oder konvex sein, um einen dichten Schluss der Saugglocke 11 mit dem Myokard/Nervenplexus 3 zu gewährleisten.

Ebenso kann im Sinne der Erfindung eine Ansaugeinrichtungen in Form einer Saugröhre mit einem zentralen Unterdrucklumen 12 (z.B. in Hockeyschlägerform zum erreichen des inferioren interatrialen Plexus 3) und zwei Elektroden 2 an der Kopfseite zur epikardialen Nervenstimulation dienen (vgl. Figur **16**).

Eine alternative Variante sieht die Fixation von epikardialen Stimulationspolen durch einen Fibrinkleber-Spritzpistole vor (vgl. Figur **17**). Die Spritzpistole besteht aus zwei Halbröhren, die zusammengesetzt eine schlussdichte Rundröhre in Form eines Hockeyschlägers bilden. An der Kopfseite des kurzen Armes dieser Röhre sind 2 runde Haltezwingen eingelassen, die 2 metallene Stifte halten, an deren distalen (außerhalb der Röhre befindlichen) Ende jeweils eine plattenförmige Stimulationspol 2 angebracht ist. Am proximalen Ende der Stifte sind jeweils die elektrischen Leiter angebracht, die an der Kopfseite des langen Röhrenarms wieder aus der Röhre austreten. Zunächst wird die zusammengesetzte Röhre mit den am Kopfteil des kurzen Röhrenarms austretenden Stimulationspolen 2 als manuell führbare Stimulationselektrode 1 benutzt, um den effektiven Stimulationspol 2 durch probatorische elektrische Stimulation zu identifizieren. Nach dem Auffinden eines effektiven Stimulationpunktes erfolgt die Fixierung der Stimulationspole 2. Dazu sind neben den Stifthalte-Zwingen mehrere Öffnungen an der Kopfseite des kurzen Röhrenarms angebracht, die jeweils durch Zufuhrkanäle in Form von tubulären Elementen 13 mit einem kleinen Reservoir am langen Röhrenende verbunden sind. Mit einer außen am Reservoir konnektierten Spritze kann dann z.B. Fibrinkleber durch die tubulären Elemente 13 und die Mündungsöffnungen am Kopfteil des kurzen Arms der Röhre gespritzt werden, sodass die auf dem Nervenplexus 3 platzierten Stimulationspole 2 vollständig von Fibrinkleber umgeben sind. Nach kurzem Anhärten des Fibrinklebers werden die Halbröhren auseinandergeklappt und entfernt, sodass die Stimulationspole 2 mit den Stiften und angeschlossenen elektrischen Leitern mittels Fibrinkleber auf dem Nervenplexus 3 "festgeschweißt" sind.

Diese Ausführungsform eignet sich besonders für die Fixierung von Stimulationspolen 2 an schwer zugänglichen Nervenstimulationsorten wie z.B. der inferiore interatriale Plexus oder der Nervenplexus zwischen der rechten Pulmonalarterie, und der Basis der Aorta sowie oberen Hohlvene.

Eine weitere Variante sieht eine kleine Plattform 14 vor, in der zwei oder mehrere Löcher eingelassen sind, durch die zwei Stimulationspole 2 an Stiften 1 durchgeschoben werden können (vgl. Figur **18**). Eine Schraube 15 an oder oberhalb der Einlassöffnung ermöglicht eine Fixierung des Elektrodenstiftes 1, sodass unterschiedliche Längen des Elektrodenstiftes 1 unterhalb der Plattform 14 erzielt werden können. Die Plattform 14 selbst weist zwei oder mehrere Ansaugeinrichtungen in Form von Saugnäpfen 16 auf, mit denen sie auf dem epikardialen Nervenplexus 3 positioniert wird. Nach Anlage eines Unterdrucks an die Saugnäpfe 16 wird die Plattform 14 über dem Nervenplexus 3 stabilisiert und fixiert. Die Stimulationsstifte 1 werden dann durch die Einlassöffnungen soweit vorgeschoben, bis sie den Nervenplexus 3 berühren und alsdann durch die Arretierschrauben an der Plattform 14 in dieser Stellung fixiert.

Eine andere Ausführungsvariante sieht eine Schraubelektrode 1 vor, die im epikardialen Nervenplexus 3 verankert wird. Die Schraubelektrode 1 weist eine elektrisch aktive Spitze 2 auf sowie einen zweiten elektrischen Stimulationspol in Form einer Ringelektrode 2 unmittelbar hinter der Spitze 2. Die Schraube weist typischerweise 3-4 (2-20) Windungen auf. Durch Einschrauben in den Nervenplexus 3 kommt der unmittelbar hinter der Schraube befindliche Elektrodenring in Kontakt mit dem epikardialen Nervenplexus 3, sodass eine bipolare elektrische Stimulation des Nervenplexus 3 gewährleistet ist (vgl. Figur **19**).

Die Nervenstimulationselektrode kann alternativ aus einem flexiblen Elektrodenkatheter 1 bestehen, in dessen Verlauf ein oder mehrere zirkuläre Stimulationspole 2 angebracht sind (Elektrodenlänge 2-5 mm, Interelektrodenabstand 2-10 mm). Zwischen den Stimulationspolen 2 sind Einlassöffnungen 17 angebracht, die über eine Verbindungslumen 18 miteinander kommunizieren und an die ein Unterdruck angelegt werden kann. Die Einlassöffnungen 17 können auch sogenannte Lippen 19 zur Erleichterung des Kontakts mit dem Epikard aufweisen. Durch Anlage des Unterdrucks wird der Katheter auf dem epikardialen Nervenplexus 3 fixiert. (vgl. Figur **20**)

Bei allen genannten Ausführungsformen sind die Stimulationspole über elektrisch leitende Drähte mit einer Stimulationseinheit verbunden. Die Stimulationseinheit besteht aus einer Impulserzeugungseinheit und einer Starteinheit.

Die Neurostimulationseinrichtung umfasst weiterhin eine Impulserzeugungseinheit 21, deren Betriebsweise im folgenden unter Bezugnahme auf Figur 21 näher erläutert wird. Bei der Impulserzeugungseinheit 21 handelt es sich bevorzugt um einen Spannungsgenerator, der in der Lage ist, elektrische Stimulationspulse zu erzeugen. Die Impulsdauer kann zwischen 0 und 20 ms liegen (typischerweise 0.05 bis 5 ms) und die Stimulationsfrequenz zwischen 0 bis 1000 Hz (typischerweise 2-100 Hz). Die Impulsform kann mono-, bi-, oder triphasisch sein. Die Stimulationsspannung kann zwischen 1 und 100 V liegen. Grundsätzlich ist eine jeweils kontinuierliche epikardiale/extravaskuläre Stimulation der Nervenplexus vorgesehen. Im Einzelfall kann aber eine intermittierende, gepulste Nervenstimulation in der atrialen/ventrikulären Refraktärzeit notwendig werden, um eine atriale/ventrikuläre elektrische myokardiale Stimulation des unter den Nervenplexus liegenden Myokards zu verhindern. Hierzu werden kurze Salven hochfrequenter elektrischer Stimuli (Frequenz: 1-1000 Hz, typischerweise 200 Hz) gekoppelt an die atriale P-Welle oder ventrikuläre R-Zacke abgegeben. Das Kopplungsintervall beträgt typischerweise 20 ms kann aber jeden Wert zwischen 0-100 ms annehmen. Die atriale oder ventrikuläre myokardiale Depolarisation sowie die sich anschließende Refraktärphase des Herzens wird über eine mit der Steuereinrichtung 19 verbundene erste Erfassungseinrichtung 28 erfasst. Dies geschieht entweder über die Nervenstimulationspole 2, die als Sensing-Elekrode dienen können, erfaßt oder durch endokardial oder epikardial plazierte atriale und/oder ventrikuläre Sensingelektroden. Alternativ kann auch eine Triggerung der gepulsten Nervenstimulation auf die P-Welle oder R- Zacke im Oberflächen EKG erfolgen. Die atrialen/ventrikulären Sensing-Signale werden ebenfalls benötigt, um die Intensität der Nervenstimuli an die jeweilige atriale/ventrikuläre Frequenz anzupassen. Die atrialen/ventrikulären Signale werden daher an die Steuereinrichtung 19 weitergeleitet, welche die Impulserzeugungseinheit dann in Abhängigkeit vom Zustand der ersten Erfassungseinrichtung betätigt bzw. ansteuert.

Die Steuereinrichtung 19 kann noch mit einer zweiten Erfassungseinheit 19.2 verbunden sein, die wiederum mit einer oder mehreren Messsonden verbunden ist. Die zweite Erfassungseinheit 30 dient hier der Erfassung des Herzzeitvolumens. Sie kann bei anderen Varianten auch zur Erfassung von anderen biologischen oder mechanischen Messgrößen, wie Herzfrequenz, Blutdruck, Sauerstoffpartialdruck, Repolarisationszeiten, Veränderungen der Erregungsrückbildung des Herzens, Körpertemperatur sowie von mechanischen Bewegungen wie Veränderung von Positionen eines Operationsroboterarmes oder des Operateurs. Eine auf die Erfassungsgrößen ansprechende Starteinheit der Steuereinrichtung 19 setzt die Impulserzeugungseinheit 21 in Betrieb, sobald die jeweilige Messgröße einen bestimmten Grenzwert unter-/überschreitet. Eine Modifikation der Starteinheit sieht vor, dass der Operateur anhand der Bewegung des Herzens bzw. der für notwendig erachteten Immobilisation des Herzens über einen (Fuß-)Schalter 26 den Beginn, die Dauer sowie die Intensität der Nervenstimulation steuert. So kann, ähnlich eines Gaspedals beim Auto, durch unterschiedlichen Fußdruck, der auf einen Fußschalter ausgeübt wird, das Ausmaß der Nervenstimulation und Bradykardisierung jederzeit den operationstechnischen Notwendigkeiten angepasst werden. In Abhängigkeit der zugrundeliegenden Herzpumpfunktion geht eine Bradykardisierung trotz Erhöhung des Schlagvolumens ab einem gewissen Grad mit einer Verminderung des Herzzeitvolumens einher. Um eine kritische Minderdurchblutung während der Nervenstimulation zu verhindern, werden biologische Parameter wie das Herzzeitvolumen, der arterielle Sauerstoffpartialdruck oder der arterielle/zentrale Venendruck dem Operateur angezeigt, sodass er das Ausmaß und die Dauer der Bradykardisierung den hämodynamischen Notwendigkeiten anpassen kann. In einer Variante der Erfindung wird die Nervenstimulationsintensität durch die Steuereinrichtung 19 automatisch vermindert, falls das Herzzeitvolumen unter einen unteren Grenzwert fällt.

Neben der Erfindung, die eine alleinige parasympathische Nervenstimulation zur Erleichterung einer Operation am schlagenden Herzen beschreibt, sieht eine Variante der Erfindung eine Kombination einer Neurostimulationseinheit mit Systemen zur lokalen mechanischen Stabilisierung/Immobilisation des myokardialen Areals in unmittelbarer Nähe eines Herzkranzgefäßes vor. Die durch Neurostimulation erzielte elektrische Immobilisation des Herzens wirkt hierbei synergistisch zur lokalen Immobilisation durch Stabilisatoren.

In einer anderen bevorzugten Ausführung ist die Neurostimulationseinrichtung mit einer Pumpeinrichtung in Form einer intravaskulären oder parakardialen Blutpumpe oder einer extrakorporalen Blutpumpe als Bestandteil einer Bewegungsreduktionseinrichtung kombiniert. Die Neurostimulationseinrichtung 20, welche die Stimulationselektrode 1 und die Impulserzeugungseinheit 21 umfasst, ist mit einer Steuereinrichtung 19 verbunden. Dasselbe gilt für die Bewegungsreduktionseinrichtung 22, welche die Blutpumpe 22 und deren Pumpensteuerung 24 umfasst. Die Blutpumpe 22 soll einerseits das durch die Bradykardie verminderte Herzzeitvolumen aufrechterhalten, zum anderen das durch die Bradykardie erhöhte Schlagvolumen eines einzelnen Herzschlages, was zu einer vermehrten Wandbewegung während einer Einzelkontraktion führt, entlasten. Zu diesem Zweck erhöht die Steuereinrichtung 19 in einem ersten Betriebsmodus automatisch den Anteil des Herzzeitvolumens, der von der Pumpe getragen wird, sobald die Herzfrequenz durch Nervenstimulation vermindert wird. Mit anderen Worten steuert die Steuereinrichtung 19 die Bewegungsreduktionseinrichtung 22 in diesem Betriebsmodus in Abhängigkeit von der Stimulationsart und insbesondere der Stimulationsintensität. Darüber hinaus sieht eine Modifikation des kombinierten Pump-/Nervenstimulationssystems in einem zweiten Betriebsmodus die getrennte, manuelle Steuerung des Neurostimulators 20 und der Pumpe 23 vor. So kann der Operateur z.B. mittels zweier Fußschalter je nach den Erfordernissen der Operation das Ausmaß der mechanischen Entlastung durch die Blutpumpe 23 und/oder der elektrischen Immobilisation durch Nervenstimulation wählen. Hierbei wird der jeweilig minimal notwendige Grad der Pumpenaktivität bzw. maximal verfügbare Grad der Bradykardie durch die programmierten unteren Grenzen des Herzzeitvolumens bestimmt. So kann beispielsweise der Grad der Bradykardie durch Neurostimulation nicht weiter gesteigert werden, wenn bei maximaler Pumpenaktivität das minimale Herzzeitvolumen unterschritten wird. Eine derartige Kombination einer Neurostimulation mit einer parakardialen Pumpe eignet sich im Speziellen auch für die Herzunterstützungstherapie über Wochen und Monate, bei denen zur Volumenentlastung und Erholung des Herzens eine Parakardialpumpe 23 implantiert wird. Da eine Frequenzsenkung des Herzens zur Reduktion des myokardialen Sauerstoffverbrauchs beiträgt kann die vorgesehene Erfindung durch eine Herzfrequenzsenkung zur Erholung des Herzmuskelgewebes beitragen. In diesem Fall werden die Nervenstimulationselektroden 1 (semi-) permanent in den Nervenplexus 3 verankert, z.B. durch Schraub oder Klemmmechanismen an der Spitze der Elektroden 1. Die Elektroden werden dann mit einem (chronisch) implantierbaren Neurostimulator, der eine Batterie und entsprechende Software Komponenten zur kardialen Neurostimulation enthält. Ein derartiges Gerät ist beispielweise in US 2002/0026222 A1 beschrieben. Bei vorteilhaften Varianten der Erfindung ist eine mit der Steuereinrichtung 19 verbundene Umschalteinrichtung 19.1 zum Umschalten zwischen dem ersten und zweiten Betriebsmodus vorgesehen.

Eine gesonderte Ausführungsform der Kombination von Neurostimulation und intra-/parakardialer Blutpumpe 23 sieht die Verlängerung der in der proximalen Pulmonalarterie (Arteria pulmonalis communis) befindlichen Auslassöffnung der intrakardialen Blutpumpe 23 um eine Neurostimulationselektrode 1 vor. Hierbei soll die Neurostimulationselektrode 1 über ein separates Lumen an der intrakardialen Pumpe 23 in die rechte (linke) Pulmonalarterie geführt werden, um dort transvaskulär parasympathische Nerven 3, die den Sinus- und AV-Knoten innervieren, elektrisch zu stimulieren **(vgl. Figur 21)**. Alternativ kann auch eine fest mit der Blutpumpe verbundene Stimulationselektrode als Verlängerung der Blutpumpe in der rechten (linken) Pulmonalarterie positioniert werden.

Bei einer speziellen Ausführungsform weist die Auslassöffnung der Blutpumpe selbst eine Stimulationselektrode 2 auf. Die Auslassöffnung der Blutpumpe 23 wird hierbei in die rechte (linke) Pulmonalarterie vorgeführt und durch Insufflation eines Ballons 25 reversibel im Gefäß fixiert. Der Ballon 25 okkludiert hierbei das Gefäß nicht, sondern weist Auslassöffnungen zur Aufrechterhaltung des Pulmonalarterienflusses auf. Der Ballon 25 weist weiterhin an seiner Zirkumferenz zwei Stimulationspole 2 auf, die durch Insufflation mit der Pulmonalarterienwand in Verbindung kommen und über die außen auf der Pulmonalarterie liegenden Nerven 3 transvaskulär elektrisch stimuliert werden können. Um bei Vorführen der Auslassöffnung in eine der beiden Pulmonalarterien eine Perfusion auch der kontralateralen Pulmonalarterie zu gewährleisten, besitzt der Auslassschlauch der Blutpumpe 23 im Bereich des Abgangs der kontralateralen Lungenarterie eine weitere seitliche Auslassöffnung (vgl. Figur **22**).

Beispielhaft wird im Folgenden der Regelkreis für eine kombinierte elektrische und mechanische Immobilisation beschrieben **(vgl. Figur 21 und 23).** Der Operateur wählt mit einer mit der Steuereinrichtung 19 verbunden ersten Vorgabeeinrichtung 26 in Form eines einem (Fuß-) Schalters das Ausmaß der gewünschten elektrischen Immobilisation (=Herzfrequenzverlangsamung) aus. Entsprechend zuvor erstellter Dosis-Wirkungskurven wird zur Erreichung der Zielgröße Herzfrequenz eine Nervenstimulationsintensität bereitgestellt. Dieser Verlauf von Stimulationsdosis und Immobilisierungswirkung wird in einem entsprechend repräsentativen Patientendatensatz in einer mit der Steuereinrichtung 19 verbunden zweiten Vorgabeeinrichtung 27 gespeichert. Die Steuereinrichtung beeinflusst die der Neurostimulationseinrichtung 20 dann in nachfolgend beschriebener Weise in Abhängigkeit von dem Patientendatensatz. Hierbei wird parallel der Herzrhythmus (Sinusrhythmus oder z.B. Vorhofflimmern) verifiziert und der entsprechende epikardiale Nervenstimulationsort gewählt (Sinusrhythmus: ventraler rechtsatrialer Plexus, Vorhofflimmern: inferiorer interatrialer Plexus). Die erzielte Herzfrequenz-(veränderung) wird von einer mit der Steuereinrichtung 19 verbundenen Herzfrequenz-Erfassungseinrichtung 28 gemessen. Sollte die tatsächliche Herzfrequenz von der Zielfrequenz abweichen, die über eine mit der Steuereinrichtung 19 verbundene dritte Vorgabeeinrichtung 29 vorgegeben ist, wird durch eine Rückkopplung die Nervenstimulationsintensität automatisch erhöht/erniedrigt. Derartige Abweichungen einer individuellen patientenspezifischen Dosis-Wirkungskurve werden erfasst und als Korrekturfaktors an die Regeleinheit der Neurostimulationseinrichtung übermittelt, sodass eine individuelle Dosis-Wirkungskurve für den Patienten erstellt wird. Über eine mit der Steuereinrichtung 19 verbundenen Herzzeitvolumen-Erfassungseinrichtung 30 in Form einer hämodynamischen Erfassungseinheit wird kontinuierlich das Schlagvolumen bzw. Herzzeitvolumen gemessen und zusammen mit der Herzfrequenz an eine Vergleichseinheit 19.1 der Steuereinrichtung 19 weitergeleitet. Diese vergleicht das Ist-Herzzeitvolumen (HZV) mit dem durch eine mit der Steuereinrichtung 19 verbundene vierte Vorgabeeinrichtung 31 vorgegebenen Soll-HZV. Da durch die Frequenzverlangsamung das Herzzeitvolumen sinken kann, wird bei Unterschreiten eines kritischen Herzzeitvolumens das Herzzeitvolumen, das durch eine Parakardialpumpe getragen wird, gesteuert durch die Steuereinrichtung 19 erhöht. Umgekehrt wird das Pumpen-HZV durch die Steuereinrichtung 19 automatisch verringert, wenn die Herzfrequenz ansteigt. Sollte bei einem gegebenen Ausmaß einer elektrischen Immobilisation (Bradykardie) zusätzlich eine mechanische Immobilisation vom Operateur erwünscht sein, kann zur Volumenentlastung des Herzens auch direkt vom Operateur durch entsprechendes einwirken auf die Steuereinrichtung 19 das von der Parakardialpumpe 23 beigesteuerte HZV erhöht werden. In die Definition der Soll-HZV Grenzen gehen patientenspezifische Parameter ein (z.B. Komorbidität wie cerebrale Gefäßverengungen, Nierenfunktion, basale Herzpumpleistung etc.). Ein derartiges System ermöglicht dem Operateur die notwendige freie Wahl des Grades und der Dauer der elektrischen Immobilisation ohne dass der Patient durch ein konsekutiv vermindertes Herzzeitvolumen kompromittiert wäre.

Mit anderen Worten beeinflusst die Steuereinrichtung 19 sowohl den Betriebszustand der Neurostimulationseinrichtung 20 als auch der Bewegungsreduktionseinrichtung 22 in Abhängigkeit von den Vorgaben der ersten bis vierten Vorgabeeinrichtung 26, 27, 29 und 31 sowie den Betriebszuständen der Herzfrequenz-Erfassungseinrichtung 28 als auch der Herzzeitvolumen-Erfassungseinrichtung 30.

Bei einer Ausführungsform mit alleiniger elektrischer Immobilisation des Herzens durch kardiale Neurostimulation wird bei Unterschreiten kritischer Untergrenzen des Herzzeitvolumen die Frequenzverlangsamung durch Neurostimulation abgeregelt **(vgl. Figur 24)**.

## Patentansprüche

1. Vorrichtung zum vorübergehenden Senken der Herzbewegung während einer Operation, insbesondere einer Herzoperation, mit einer Neurostimulationseinrichtung (20) zur Stimulation die Herzfrequenz verlangsamender Nerven (3), die wenigstens eine Elektrodeneinrichtung (1) mit wenigstens einem Stimulationspol (2) umfasst, wobei eine mit der Neurostimulationseinrichtung (20) verbundene Steuereinrichtung (19) vorgesehen ist, die eine erste Vorgabeeinrichtung (26) zur Vorgabe eines elektrischen Immobilisierungsgrades des Herzens aufweist und die zum patientenspezifischen Beeinflussen des Betriebszustandes der Neurostimulationseinrichtung (20) in Abhängigkeit von dem vorgegebenen elektrischen Immobilisierungsgrad ausgebildet ist, **dadurch gekennzeichnet, dass** die erste Vorgabeeinrichtung zur im Betrieb variablen Vorgabe des elektrischen Immobilisierungsgrades ausgebildet ist.

2. Vorrichtung nach Anspruch 1, **dadurch gekennzeichnet, dass** der Stimulationspol (2) der Elektrodeneinrichtung (1) eine wirksame Stimulationsfläche von 1 bis 100 mm², vorzugsweise von 4 bis 9 mm², aufweist.

3. Vorrichtung nach Anspruch 1 oder 2, **dadurch gekennzeichnet, dass** die Elektrodeneinrichtung (1) für eine bipolare Simulation wenigstens zwei Stimulationspole (2) aufweist, die voneinander beabstandet angeordnet sind, wobei der Abstand zwischen den Stimulationspolen (2) insbesondere im wesentlichen 2 bis 10 mm beträgt.

4. Vorrichtung nach einem der vorhergehenden Ansprüche, **dadurch gekennzeichnet, dass** die Elektrodeneinrichtung (1) zum Einführen, insbesondere Einstechen, in einen Nervenplexus (3) ausgeführt ist.

5. Vorrichtung nach einem der vorhergehenden Ansprüche, **dadurch gekennzeichnet, dass** die Elektrodeneinrichtung (1) wenigstens eine Arretiervorrichtung (5, 7, 11, 12, 13, 16, 17) zum Fixieren der Elektrodeneinrichtung (1) an einem Nervenplexus (3) und/oder einem Blutgefäß aufweist.

6. Vorrichtung nach Anspruch 5, **dadurch gekennzeichnet, dass** die Arretiervorrichtung
- wenigstens eine zangenartig ausgebildete Befestigungseinrichtung mit wenigstens zwei Zangenarmen (7) zum Festklemmen der Elektrodeneinrichtung (1) an einem Nervenplexus (3) und/oder einem Blutgefäß aufweist, wobei wenigstens ein Stimulationspol (2) im Bereich des freien Endes eines Zangenarms (7) angeordnet ist
oder
- wenigstens eine Ansaugeinrichtung (11, 12, 16) mit wenigstens einer Ansaugöffnung zum Befestigen der Elektrodeneinrichtung am menschlichen Gewebe mittels Unterdruck aufweist, wobei der Stimulationspol (2) insbesondere im Bereich der Ansaugöffnung angeordnet ist,
oder
- wenigstens einen Zufuhrkanal (13) für Gewebeklebstoff, insbesondere Fibrinklebstoff, mit wenigstens einer Mündungsöffnung zum Festkleben der Elektrodeneinrichtung (11) im Bereich der Mündungsöffnung umfasst, wobei der Stimulationspol (2) insbesondere im Bereich der Mündungsöffnung angeordnet ist.

7. Vorrichtung nach einem der vorhergehenden Ansprüche, **dadurch gekennzeichnet, dass** die Elektrodeneinrichtung (1) nach Art einer Schraubelektrode ausgebildet ist.

8. Vorrichtung nach einem der vorhergehenden Ansprüche, **dadurch gekennzeichnet, dass** die Elektrodeneinrichtung (1) zum Verhindern einer unerwünschten Stimulation von Herzgewebe, insbesondere von Myokardgewebe, wenigstens eine dem Stimulationspol (2) zugeordnete Abschirmeinrichtung (6) aufweist.

9. Vorrichtung nach einem der vorhergehenden Ansprüche, **dadurch gekennzeichnet, dass** die Neurostimulationseinrichtung (20) eine mit der Elektrodeneinrichtung (1) verbundene Impulserzeugungseinheit (21) aufweist, die zur Ansteuerung weiterhin mit der Steuereinrichtung (19) verbunden ist.

10. Vorrichtung nach Anspruch 9, **dadurch gekennzeichnet, dass** die Impulserzeugungseinheit (21) zur Erzeugung von Impulsen mit einer Dauer zwischen 0 und 20 ms, vorzugsweise von 0,05 bis 5 ms, und/oder mit einer Stimulationsfrequenz zwischen 0 und 1000 Hz, vorzugsweise von 2 bis 100 Hz, und/oder einer Stimulationsspannung zwischen 1 und 100 V ausgebildet ist.

11. Vorrichtung nach Anspruch 9 oder 10, **dadurch gekennzeichnet, dass** die Impulserzeugungseinheit (21) zur kontinuierlichen Stimulation ausgebildet ist oder dass die Impulserzeugungseinheit (21) zur intermittierenden Stimulation unter Erzeugung kurzer Salven hochfrequenter Impulse ausgebildet ist.

12. Vorrichtung nach Anspruch 11, **dadurch gekennzeichnet, dass** eine mit der Steuereinrichtung (19) verbundene erste Erfassungseinrichtung (28) zum Erfassen der Refraktärphase des Herzens vorgesehen ist und die Steuereinrichtung (19) zum Betätigen Impulserzeugungseinheit (21) in Abhängigkeit vom Zustand der ersten Erfassungseinrichtung (28) ausgebildet ist, wobei die erste Erfassungseinrichtung (28) insbesondere wenigstens eine Sensing-Elektrode umfasst, die von der Elektrodeneinrichtung (1) gebildet ist.

13. Vorrichtung nach einem der vorhergehenden Ansprüche, **dadurch gekennzeichnet, dass** wenigstens eine mit der Steuereinrichtung (19) verbundene zweite Erfassungseinrichtung (30) zur Erfassung wenigstens einer biologischen oder mechanischen Messgröße vorgesehen ist und dass die Steuereinrichtung (19) zum Beeinflussen der Neurostimulationseinrichtung (20) in Abhängigkeit vom Zustand der zweiten Erfassungseinrichtung (30) ausgebildet ist.

14. Vorrichtung nach einem der vorhergehenden Ansprüche, **dadurch gekennzeichnet, dass** eine mit der Steuereinrichtung (19) verbundene Bewegungsreduktionseinrichtung (22) vorgesehen ist, wobei die Steuereinrichtung (19) zum Beeinflussen des Betriebszustands der Bewegungsreduktionseinrichtung (22) ausgebildet ist.

15. Vorrichtung nach Anspruch 14, **dadurch gekennzeichnet, dass** die Steuereinrichtung (19) in einem ersten Betriebsmodus zum Beeinflussen des Betriebszustands der Bewegungsreduktionseinrichtung (22) in Abhängigkeit vom Betriebszustand der Neurostimulationseinrichtung (20) ausgebildet ist und/oder in einem zweiten Betriebsmodus zum getrennten Beeinflussen des Betriebszustands der Bewegungsreduktionseinrichtung (22) und der Neurostimulationseinrichtung (20) ausgebildet ist, wobei die Steuereinrichtung (19) insbesondere eine Umschalteinrichtung (19.1) zum Umschalten zwischen dem ersten und zweiten Betriebsmodus umfasst.

16. Vorrichtung nach Anspruch 14 oder 15, **dadurch gekennzeichnet, dass** die Bewegungsreduktionseinrichtung (22) eine Pumpeinrichtung (23) zur Unterstützung der Herzfunktion und/oder eine Stabilisationseinrichtung zur Stabilisierung der Herzwand umfasst.

17. Vorrichtung nach einem der Ansprüche 14 bis 16, **dadurch gekennzeichnet, dass** die Steuereinrichtung (19) derart ausgebildet ist, dass sie den Betriebszustand der Bewegungsreduktionseinrichtung (22) in Abhängigkeit von der Stimulationsart und/oder der Stimulationsintensität der Neurostimulationseinrichtung (20) steuert.

18. Vorrichtung nach einem der Ansprüche 14 bis 17, **dadurch gekennzeichnet, dass** eine mit der Steuereinrichtung (19) verbundene zweite Vorgabeeinrichtung (27) zum Speichern wenigstens eines patientenspezifischen für den Verlauf von Stimulationsdosis und Immobilisierungswirkung repräsentativen Patientendatensatzes vorgesehen ist und die Steuereinrichtung (19) zur Beeinflussung der Neurostimulationseinrichtung (20) in Abhängigkeit von dem Patientendatensatz ausgebildet ist.

19. Vorrichtung nach einem der Ansprüche 14 bis 18, **dadurch gekennzeichnet, dass** eine mit der Steuereinrichtung (19) verbundene Herzfrequenz-Erfassungseinrichtung (28) zum Erfassen eines für die Ist-Herzfrequenz repräsentativen Herzfrequenz-Signals vorgesehen ist und die Steuereinrichtung (19) zur Beeinflussung der Neurostimulationseinrichtung (20) und/oder der Bewegungsreduktionseinrichtung (22) in Abhängigkeit vom Zustand der Herzfrequenz-Erfassungseinrichtung (28) ausgebildet ist.

20. Vorrichtung nach Anspruch 19, **dadurch gekennzeichnet, dass** eine mit der Steuereinrichtung (19) verbundene dritte Vorgabeeinrichtung (29) zur Vorgabe einer Soll-Herzfrequenz vorgesehen ist und die Steuereinrichtung (19) zur Beeinflussung der Neurostimulationseinrichtung (20) und/oder der Bewegungsreduktionseinrichtung (22) in Abhängigkeit vom Zustand der Herzfrequenz-Erfassungseinrichtung (28) und der dritten Vorgabeeinrichtung (29) ausgebildet ist.

21. Vorrichtung nach einem der Ansprüche 14 bis 20, **dadurch gekennzeichnet, dass** eine mit der Steuereinrichtung (19) verbundene Herzzeitvolumen-Erfassungseinrichtung (30) zum Erfassen eines für das Ist-Herzzeitvolumen repräsentativen Herzzeitvolumen-Signals vorgesehen ist und die Steuereinrichtung (19) zur Beeinflussung der Neurostimulationseinrichtung (20) und/oder der Bewegungsreduktionseinrichtung (22) in Abhängigkeit vom Zustand der Herzzeitvolumen-Erfassungseinrichtung (30) ausgebildet ist.

22. Vorrichtung nach Anspruch 21, **dadurch gekennzeichnet, dass** eine mit der Steuereinrichtung (19) verbundene vierte Vorgabeeinrichtung (31) zur Vorgabe eines Soll-Herzzeitvolumens vorgesehen ist und die Steuereinrichtung (19) zur Beeinflussung der Neurostimulationseinrichtung (20) und/oder der Bewegungsreduktionseinrichtung (22) in Abhängigkeit vom Zustand der Herzzeitvolumen-Erfassungseinrichtung (30) und der vierten Vorgabeeinrichtung (31) ausgebildet ist.

23. Vorrichtung nach einem der Ansprüche 14 bis 22, **dadurch gekennzeichnet, dass** die Bewegungsreduktionseinrichtung (22) eine Pumpeinrichtung (23) zur Unterstützung der Herzfunktion umfasst und die Elektrodeneinrichtung (1) der Neurostimulationseinrichtung (20) an der Pumpeinrichtung (23) angeordnet ist, wobei der Stimulationspol (2) insbesondere an der Pumpeinrichtung (23) angeordnet ist.

## Claims

1. A device for temporarily reducing the movement of the heart during surgery, in particular during cardiac surgery, with a neurostimulation device (20) for stimulating nerves (3) that slow down the heart rate, comprising at least one electrode device (1) with at least one stimulation pole (2), wherein a control unit (19) connected to the neurostimulation device (20) is provided, comprising a first preselection device for preselecting a degree of electric immobilization of the heart and arranged for patent specifically influencing the operation mode of the neurostimulation device (20) as a function of said preselected degree of electric immobilization, **characterized in that** said first input device is arranged for variably preselecting said degree of electric immobilization during operation.

2. The device according to claim 1, **characterized in that** the stimulation pole (2) of the electrode device (1) has an effective stimulation area of 1 to 100 mm², preferably 4 to 9 mm².

3. The device according to claim 1 and 2, **characterized in that** the electrode device (1) has at least two stimulation poles (2) for bipolar stimulation which are arranged spatially separate, wherein, in particular, the distance between the stimulation poles (2) amounts to essentially 2 to 10 mm.

4. The device according to one of the preceding claims, **characterized in that** the electrode device (1) is designed for inserting into, in particular puncturing, a nerve plexus (3).

5. The device according to one of the preceding claims, **characterized in that** the electrode device (1) has at least one locking device (5, 7, 11, 12, 13, 16, 17) for securing the electrode device (1) on a nerve plexus (3) and/or on a blood vessel.

6. The device according to claim 5, **characterized in that** the locking device
- has at least one fastening device designed like a forceps with at least two forceps arms (7) for securely clamping the electrode device (1) on a nerve plexus (3) and/or a blood vessel, whereby at least one stimulation pole (2) is arranged in the area of the free end of a forceps arm (7)
or
- has at least one suction device (11, 12, 16) having at least one suction opening for fastening the electrode device to human tissue by means of a negative pressure, wherein the stimulation pole (2) is situated, in particular, in the area of the suction opening
or
- has at least one supply channel (13) for tissue adhesive, in particular fibrin adhesive, with at least one mouth opening for securing the electrode device (11) with adhesive in the area of the mouth opening, wherein the stimulation pole (2) is situated, in particular, in the area of the mouth opening.

7. The device according to one of the preceding claims, **characterized in that** the electrode device (1) is designed in the manner of a screw electrode.

8. The device according to one of the preceding claims, **characterized in that** the electrode device (1) has a shielding device (6) associated to the stimulation pole (2) to prevent unwanted stimulation of cardiac tissue, in particular myocardial tissue.

9. The device according to one of the preceding claims, **characterized in that** the neurostimulation device (20) has a pulse generating unit (21) that is connected to the electrode device (1) and is also connected to the control unit (19) for triggering purposes.

10. The device according to claim 9, **characterized in that** the pulse generating unit is designed for generating pulses with a duration between 0 and 20 ms, preferably 0.05 to 5 ms, and/or with a stimulation frequency between 0 and 1000 Hz, preferably 2 to 100 Hz, and/or a stimulation voltage between 1 and 100 V.

11. The device according to claim 9 or 10, **characterized in that** the pulse generating unit (21) is designed for continuous stimulation or that the pulse generating unit (21) is designed for intermittent stimulation, generating short bursts of highfrequency pulses.

12. The device according to claim 11, **characterized in that** a first detection unit (28) connected to the control unit (19) is provided for detecting the refractory phase of the heart, and the control unit (19) is designed for operating the pulse generating unit (21) as a function of the state of the first detection unit (28), wherein, in particular, the first detection unit (28) includes at least one sensing electrode which is formed by the electrode device (1).

13. The device according to one of the preceding claims, **characterized in that** at least one second detection unit (30) connected to the control unit (19) is provided for detecting at least one biological or mechanical measurement value and the control unit (19) is designed for influencing the neurostimulation device (20) as a function of the state of the second detection unit (30).

14. The device according to one of the preceding claims, **characterized in that** a movement reducing device (22) connected to the control unit (19) is provided, the control unit (19) being designed for influencing the operating state of the movement reducing device (22).

15. The device according to claim 14, **characterized in that** the control unit (19) is designed for influencing, in a first operating mode, the operating state of the movement reducing device (22) as a function of the operating state of the neurostimulation device (20) and/or for separately influencing, in a second operating mode, the operating state of the movement reducing device (22) and the neurostimulation device (20), wherein, in particular, the control unit (19) includes a switching device (19.1) for switching between the first operating mode and the second operating mode.

16. The device according to claim 14 or 15, **characterized in that** the movement reducing device (22) includes a pump device (23) for supporting cardiac function and/or a stabilization device for stabilizing the cardiac wall.

17. The device according to one of claims 14 to 16, **characterized in that** the control unit (19) is designed for controlling the operating state of the movement reducing device (22) as a function of the type of stimulation and/or the stimulation intensity of the neurostimulation device (20).

18. The device according to one of claims 14 to 17, **characterized in that** a second preselection device (27) which is connected to the control unit (19) is provided for storing at least one patient-specific data record that is representative of the course of the stimulation dose and the immobilization effect, and the control unit (19) is designed for influencing the neurostimulation device (20) as a function of the patient data record.

19. The device according to one of claims 14 to 18, **characterized in that** a heart rate detection device (28) which is connected to the control unit (19) is provided for detecting a heart rate signal that is representative of the actual heart rate, and the control unit (19) is designed for influencing the neurostimulation device (20) and/or the movement reducing device (22) as a function of the state of the heart rate detection device (28).

20. The device according to claim 19, **characterized in that** a third preselection device (29) connected to the control unit (19) is provided for input of a setpoint heart rate, and the control unit (19) is designed for influencing the neurostimulation device (20) and/or the movement reducing device (22) as a function of the state of the heart rate detection device (28) and the third preselection device (29).

21. The device according to one of claims 14 to 20, **characterized in that** a cardiac output detection device (30) connected to the control unit (19) is provided for detecting a cardiac output signal that is representative of the actual cardiac output, and the control unit (19) is designed for influencing the neurostimulation device (20) and/or the movement reducing device (22) as a function of the state of the cardiac output detection device (30).

22. The device according to claim 21, **characterized in that** a fourth preselection device (31) connected to the control unit (19) is provided for preselecting a setpoint cardiac output and the control device (19) is designed for influencing the neurostimulation device (20) and/or the movement reducing device (22) as a function of the state of cardiac output detection device (30) and the fourth preselection device (31).

23. The device according to one of claims 14 to 22, **characterized in that** the movement reducing device (22) includes a pump unit (23) for supporting heart function, and the electrode device (1) of the neurostimulation device (20) is situated on the pump unit (23), wherein, in particular, the stimulation pole (2) is situated on the pump unit (23).

## Revendications

1. Procédé de réduction temporaire du mouvement du coeur pendant une opération, notamment une opération du coeur, à l'aide d'un dispositif de neurostimulation (20) servant à stimuler des nerfs (3) ralentissant la fréquence cardiaque, qui comprend au moins un dispositif à électrode (1) présentant au moins un pôle de stimulation (2), étant prévu un dispositif de commande (19) qui est raccordé au dispositif de neurostimulation (20) et qui présente un premier dispositif prescripteur (26) pour la prescription d'un degré d'immobilisation électrique du coeur et qui est conçu pour influer de manière spécifique au patient sur l'état de fonctionnement du dispositif de neurostimulation (20) en fonction du degré d'immobilisation électrique du coeur prescrit, **caractérisé en ce que** le premier dispositif prescripteur est conçu pour faire une prescription variable en cours de fonctionnement du degré d'immobilisation électrique.

2. Dispositif selon la revendication 1, **caractérisé en ce que** le pôle de stimulation (2) du dispositif électrique (2) du dispositif à électrode (1) présente une surface de stimulation effective de 1 à 100 mm², de préférence de 4 à 9 mm².

3. Dispositif selon la revendication 1 ou 2, **caractérisé en ce que** le dispositif à électrode (1) présente, pour une stimulation bipolaire, au moins deux pôles de stimulation (2) qui sont disposés à distance l'un de l'autre, la distance entre les pôles de stimulation (2) s'élevant notamment sensiblement à 2 à 10 mm.

4. Dispositif selon l'une quelconque des revendications précédentes, **caractérisé en ce que** le dispositif à électrode (1) est conçu pour être introduit, notamment piqué, dans un plexus nerveux (3).

5. Dispositif selon l'une quelconque des revendications précédentes, **caractérisé en ce que** le dispositif à électrode (1) présente au moins un dispositif de blocage (5, 7, 11, 12, 13, 16, 17) pour fixer le dispositif à électrode (1) à un plexus nerveux (3) et/ou un vaisseau sanguin.

6. Dispositif selon la revendication 5, **caractérisé en ce que** le dispositif de blocage
- présente au moins un système de fixation réalisé en forme de pince et comportant au moins deux bras de pince (7) pour le blocage du dispositif à électrode (7) sur un plexus nerveux (3) et/ou un vaisseau sanguin, au moins un pôle de stimulation (2) étant disposé au niveau de l'extrémité libre d'un bras de pince (7)
ou
- présente au moins un dispositif d'aspiration (11, 12, 16) comportant au moins d'un orifice d'aspiration pour la fixation du dispositif à électrode aux tissus humains par dépressurisation, le pôle de stimulation (2) étant disposé notamment au niveau de l'orifice d'aspiration,
ou
- au moins un canal d'acheminement (13) pour de la colle à tissus, notamment de la colle de fibrine, comportant au moins un orifice d'embouchure pour le collage du dispositif à électrode (11) au niveau de l'orifice d'embouchure, le pôle de stimulation (2) étant disposé notamment au niveau de l'orifice d'embouchure.

7. Dispositif selon l'une quelconque des revendications précédentes, **caractérisé en ce que** le dispositif à électrode (1) est réalisé à la manière d'une électrode vissée.

8. Dispositif selon l'une quelconque des revendications précédentes, **caractérisé en ce que** le dispositif à électrode (1), afin d'empêcher une stimulation indésirable des tissus cardiaques, notamment des tissus du myocarde, présente au moins un dispositif faisant écran (6) associé au pôle de stimulation (2).

9. Dispositif selon l'une quelconque des revendications précédentes, **caractérisé en ce que** le dispositif de neurostimulation (20) présente une unité génératrice d'impulsions (21) qui est raccordée au dispositif à électrode (1) et qui est, pour la commande, raccordée en plus au dispositif de commande (19).

10. Dispositif selon la revendication 9, **caractérisé en ce que** l'unité génératrice d'impulsions (21) est conçue pour générer des impulsions d'une durée allant de 0 à 20 ms, de préférence de 0,05 à 5 ms, et/ou avec une fréquence de stimulation allant de 0 à 1000 Hz, de préférence de 2 à 100 Hz, et/ou une tension de stimulation allant de 1 à 100 V.

11. Dispositif selon la revendication 9 ou 10, **caractérisé en ce que** l'unité génératrice d'impulsions (21) est conçue pour une stimulation continue ou que l'unité génératrice d'impulsions (21) est conçue pour la stimulation intermittente tout en générant des salves courtes d'impulsions à haute fréquence.

12. Dispositif selon la revendication 11, **caractérisé en ce qu'**un premier dispositif d'enregistrement (28) raccordé au dispositif de commande est prévu pour enregistrer la phase réfractaire du coeur et que le dispositif de commande (19) est conçu pour actionner l'unité génératrice d'impulsions (21) en fonction de l'état du premier dispositif d'enregistrement (28), le premier dispositif d'enregistrement (28) comprenant notamment au moins une électrode de détection qui est constituée par le dispositif à électrode (1).

13. Dispositif selon l'une quelconque des revendications précédentes, **caractérisé en ce qu'**au moins un deuxième dispositif d'enregistrement (10) raccordé au dispositif de commande (19) est prévu pour l'enregistrement d'au moins une grandeur de mesure biologique ou mécanique et que le dispositif de commande (19) est conçu pour influer sur le dispositif de neurostimulation (20) en fonction de l'état du deuxième dispositif d'enregistrement (10).

14. Dispositif selon l'une quelconque des revendications précédentes, **caractérisé en ce qu'**un dispositif de réduction de mouvement (22) raccordé au dispositif de commande (19) est prévu, le dispositif de commande (19) étant conçu pour influer sur l'état de fonctionnement du dispositif de réduction de mouvement (22).

15. Dispositif selon la revendication 14, **caractérisé en ce que** le dispositif de commande (19) est conçu pour influer, dans un premier mode de fonctionnement, sur l'état de fonctionnement du dispositif de réduction de mouvement (22) en fonction de l'état de fonctionnement du dispositif de neurostimulation (20) et/ou pour influer, dans un deuxième mode de fonctionnement, séparément sur l'état de fonctionnement du dispositif de réduction de mouvement (22) et du dispositif de neurostimulation (20), le dispositif de commande (19) comprenant notamment un dispositif de commutation (19.1) pour la commutation entre le premier et le deuxième mode de fonctionnement.

16. Dispositif selon la revendication 14 ou 15, **caractérisé en ce que** le dispositif de réduction de mouvement (22) comprend un dispositif à pompe (23) pour assister la fonction cardiaque et/ou un dispositif stabilisateur pour stabiliser la paroi cardiaque.

17. Dispositif selon l'une quelconque des revendications 14 à 16, **caractérisé en ce que** le dispositif de commande (19) est réalisé de manière à contrôler l'état de fonctionnement du dispositif de réduction de mouvement (22) en fonction du type de stimulation et/ou de l'intensité de stimulation du dispositif de neurostimulation (20).

18. Dispositif selon l'une quelconque des revendications 14 à 17, **caractérisé en ce qu'**un deuxième dispositif prescripteur (27) raccordé au dispositif de commande (19) est prévu pour l'enregistrement d'au moins un ensemble de données spécifique au patient et représentatif de l'évolution de la dose de stimulation et de l'effet d'immobilisation et que le dispositif de commande (19) est conçu pour influer sur le dispositif de neurostimulation (20) en fonction de l'ensemble des données sur le patient.

19. Dispositif selon l'une quelconque des revendications 14 à 18, **caractérisé en ce qu'**un dispositif d'enregistrement de la fréquence cardiaque (28) raccordé au dispositif de commande (19) est prévu pour l'enregistrement d'un signal de fréquence cardiaque représentatif de la fréquence cardiaque actuelle et que le dispositif de commande (19) est conçu pour influer sur le dispositif de neurostimulation (20) et/ou le dispositif de réduction de mouvement (22) en fonction de l'état du dispositif d'enregistrement de la fréquence cardiaque (28).

20. Dispositif selon la revendication 19, **caractérisé en ce qu'**un troisième dispositif prescripteur (29) raccordé au dispositif de commande (19) est prévu pour la prescription d'une fréquence cardiaque théorique et que le dispositif de commande (19) est conçu pour influer sur le dispositif de neurostimulation (20) et/ou le dispositif de réduction de mouvement (22) en fonction de l'état du dispositif d'enregistrement de la fréquence cardiaque (28) et du troisième dispositif prescripteur (29).

21. Dispositif selon l'une quelconque des revendications 14 à 20, **caractérisé en ce qu'**un dispositif d'enregistrement du volume de temps cardiaque (30) raccordé au dispositif de commande (19) est prévu pour l'enregistrement d'un signal de volume de temps cardiaque représentatif du volume de temps cardiaque actuel et que le dispositif de commande (19) est conçu pour influer sur le dispositif de neurostimulation (20) et/ou le dispositif de réduction de mouvement (22) en fonction de l'état du dispositif d'enregistrement de volume de temps cardiaque (30).

22. Dispositif selon la revendication 21, **caractérisé en ce qu'**un quatrième dispositif prescripteur (31) raccordé au dispositif de commande (19) est prévu pour la prescription d'un volume de temps cardiaque théorique et que le dispositif de commande (19) est conçu pour influer sur le dispositif de neurostimulation (20) et/ou le dispositif de réduction de mouvement (22) en fonction de l'état du dispositif d'enregistrement du volume de temps cardiaque (30) et du quatrième dispositif prescripteur (31).

23. Dispositif selon l'une quelconque des revendications 14 à 22, **caractérisé en ce que** le dispositif de réduction de mouvement (22) comprend un dispositif à pompe (23) pour assister la fonction cardiaque et que le dispositif à électrode (1) du dispositif de neurostimulation (20) est disposé sur le dispositif à pompe (23), le pôle de stimulation (2) étant disposé notamment sur le dispositif à pompe (23).
